# FASCICULE DE BREVET EUROPEEN

(11) **EP 4 642 830 B1**
(45) Date de publication et mention de la délivrance du brevet: **18.11.2026**
(21) Numéro de dépôt: 23833076.5
(22) Date de dépôt: 15.12.2023
(51) Int. Cl.: C08G 63/47, C08F 220/34, C08G 63/685

(54) **MÉLANGE D'ISOCYANURATES (MÉTH)ACRYLÉS, SON PROCÉDÉ DE PRÉPARATION ET SES UTILISATIONS**
(METH)ACRYLIERTE ISOCYANURATMISCHUNG, VERFAHREN ZUR HERSTELLUNG DAVON UND VERWENDUNGEN DAVON
(METH)ACRYLATED ISOCYANURATE MIXTURE, PROCESS FOR PREPARING SAME, AND USES THEREOF

(30) Priorité: 29.12.2022 FR 2214645
(43) Date de publication de la demande: 05.11.2025
(73) Titulaire: ARKEMA France, 92800 Puteaux (FR)
(72) Inventeur: CICERON, Philippe, 60300 SENLIS (FR); DRUJON, Xavier, 60550 VERNEUIL EN HALATTE (FR); LEFORT, Alice, 60550 VERNEUIL EN HALATTE (FR); ROBIN, Elodie, 60550 VERNEUIL EN HALATTE (FR)
(74) Mandataire: Arkema Patent
(86) Numéro de dépôt international: PCT/EP2023/086025
(87) Numéro de publication internationale: WO 2024/141286

(56) Documents cités:
- JP-B2- H0 681 782
- US-B1- 6 239 189

## Description

### OBJET DE L'INVENTION

La présente invention concerne un mélange d'isocyanurates (méth)acrylés, son procédé de préparation, une composition polymérisable le comprenant et ses utilisations, notamment comme liant dans une composition polymérisable ou dans une composition pour la fabrication additive, en particulier pour l'impression d'un objet 3D ou 4D.

### ARRIERE-PLAN DE L'INVENTION

Les résines photoréticulables à base de monomères et/ou d'oligomères fonctionnalisés par des groupes (méth)acrylates, sont notamment utilisées dans la fabrication de composants par impression 3D, de revêtements pour diverses applications (notamment dans le domaine des arts graphiques), d'adhésifs et de mastics. Ces résines réagissent sous énergie UV et/ou LED pour conférer au produit final des propriétés de dureté, de flexibilité et/ou de résistance aux produits chimiques, à l'eau ou aux taches. Un monomère acrylate de ce type est commercialisé par la société Arkema (Sartomer) sous la référence SR368^{®}. Il est constitué de triacrylate de tris(2-hydroxyéthyl) isocyanurate (THEICTA). Ce monomère présente des propriétés exceptionnelles de réactivité, dureté et résistance chimique, qui lui confèrent un avantage par rapport à d'autres monomères de type polyacrylate de polyol, en particulier le triacrylate de triméthylolpropane (TMPTA), tout en conservant les propriétés de flexibilité, de tenue au solvant et de résistance aux taches des matériaux obtenus à partir du TMPTA. Le THEICTA présente toutefois l'inconvénient d'être solide, ce qui nécessite son réchauffage à plus de 52-53°C avant mise en œuvre par les formulateurs.

Des polyesters (méth)acrylates à base de tris(2-hydroxyéthyl) isocyanurate (THEIC) ont par ailleurs été décrits dans le brevet JP94081782 (JPH0681782). Ces polymères sont obtenus suivant un procédé en deux étapes, comprenant une première étape de (méth)acrylation partielle d'un mélange de polyols, dont le THEIC et le triméthylolpropane (TMP), suivie d'une étape de polycondensation avec un polyacide, dont l'acide adipique ou l'anhydride tétrahydrophtalique, pour former un polyester. Le ratio molaire diacide / polyol(s) / acide (méth)acrylique est de 1/2/4 ou 1/2/2 et le ratio THEIC/autre polyol (en particulier TMP) va de 1/3 à 3/1. L'inclusion du monomère THEIC permet de limiter l'inhibition de la polymérisation par l'oxygène de l'air sans utiliser d'additifs tels que des amines. Les présents inventeurs ont déterminé que le polymère obtenu dans ce brevet contenait une quantité de THEICTA résiduelle inférieure à 10% du poids du copolymère, plus du TMPTA issu du TMP.

Dans le même ordre d'idées, la demande de brevet CN101838377 divulgue un composant de composition photopolymérisable, obtenu à partir d'un polyol (tel que le THEIC ou le pentaérythritol), d'un acide polybasique tel que l'acide adipique et d'acide (méth)acrylique, dans un ratio molaire acide / polyol / acide (méth)acrylique de 1/2/7 dans le cas d'un diacide.

Il a toutefois été observé que certains mono- et polyesters (méth)acrylates à base de THEIC avaient tendance à former des grains à température ambiante, dus à la recristallisation du THEICTA. Les formulateurs de ces résines sont donc contraints de les réchauffer au-delà du point de fusion de ces cristaux.

Il subsiste donc le besoin de disposer d'une résine à base d'isocyanurates (méth)acrylés et qui ne présente pas de problèmes de recristallisation après au moins quatre mois à température ambiante, tout en conservant les propriétés avantageuses du THEICTA décrites précédemment.

Après d'intenses recherches, la Demanderesse a mis au point une résine qui permet de satisfaire au besoin ci-dessus, ainsi qu'un procédé de préparation de cette résine permettant d'introduire des impuretés ciblées, en quantité donnée, dans un tri(méth)acrylate de tris(hydroxyalkyle) isocyanurate (THAICT(M)A). Le produit obtenu est un mélange renfermant majoritairement du THAICT(M)A, ainsi que des produits de condensation de THAIC (méth)acrylé avec un diacide carboxylique pour former un polyester. Ce produit étant liquide, il peut être facilement mis en œuvre par les formulateurs dans la fabrication 3D ou additive ou la fabrication de revêtements ou d'adhésifs. En outre, il ne présente pas de recristallisation pendant au moins 4 mois à température ambiante, après dilution dans des monomères à haut pouvoir diluant (tels que le diacrylate de 1,6-hexanediol) ou dans des monomères visqueux de haute Tg (tels que le diacrylate de tricyclodécane diméthanol) utilisés dans ces applications. Le formulateur dispose ainsi d'une grande flexibilité quant au choix du taux de dilution, en fonction de la Tg et de la viscosité qu'il souhaite conférer à la composition photopolymérisable. Les compositions ainsi obtenues présentent en outre une bonne réactivité tandis que les produits obtenus à partir de ces compositions présentent d'excellentes propriétés en termes de dureté, de flexibilité et de résistance aux taches.

### RESUME DE L'INVENTION

L'invention a pour objet un mélange d'isocyanurates (méth)acrylés, caractérisé en ce qu'il est obtenu par réaction entre :
(a) au moins un acide dicarboxylique,
(b) au moins un monomère (méth)acrylique choisi parmi l'acide acrylique, l'acide méthacrylique, leurs anhydrides et leurs mélanges, et éventuellement au moins un acide monocarboxylique en C6-C24,
(c) au moins un isocyanurate de tris(hydroxyalkyle) et éventuellement un autre polyol,
étant entendu que le ratio molaire des groupes -COOH du constituant (a) aux groupes -OH du constituant (c) est compris entre 1:4 et 1:20, de préférence entre 1:5 et 1:15, plus préférablement entre 1:6 et 1:12.

Elle a également pour objet un procédé de préparation du mélange d'isocyanurates (méth)acrylés tel que décrit ci-dessus, caractérisé en ce qu'il comprend les étapes suivantes :
1) faire réagir tout ou partie d'un constituant (b) comprenant au moins un monomère (méth)acrylique choisi parmi l'acide acrylique, l'acide méthacrylique, leurs anhydrides et leurs mélanges et éventuellement au moins un acide monocarboxylique en C6-C24, avec un constituant (c) comprenant au moins un isocyanurate de tris(hydroxyalkyle) et éventuellement un autre polyol, dans des conditions permettant d'estérifier de 50 à 95%, de préférence de 60 à 90%, plus préférentiellement de 70 à 85%, des groupes OH du constituant (c),
2) faire réagir le produit de l'étape 1) avec un constituant (a) comprenant au moins un acide dicarboxylique et éventuellement avec le reste, le cas échéant, du constituant (b).

L'invention a encore pour objet une composition polymérisable caractérisée en ce qu'elle comprend au moins un mélange d'isocyanurates (méth)acrylés tel que décrit ci-dessus et au moins un autre composé éthyléniquement insaturé, en particulier un monomère fonctionnalisé par (méth)acrylate.

Elle a également pour objet l'utilisation d'un mélange d'isocyanurates (méth)acrylés tel que décrit ci-dessus comme liant dans une composition polymérisable ou dans une composition pour la fabrication additive, en particulier pour l'impression d'un objet 3D ou 4D.

### DESCRIPTION DETAILLEE

Dans la suite de cette description, l'expression "compris entre" s'entend comme désignant une plage de valeurs incluant les bornes citées.

L'invention porte sur un mélange d'isocyanurates (méth)acrylés particulier. Au sens de l'invention, le terme " isocyanurate (méth)acrylé " correspond à un composé ayant au moins un groupe isocyanurate et au moins un groupe (méth)acrylate.

Tel qu'utilisé ici, le terme « isocyanurate » correspond à un groupe de formule (I) suivante :

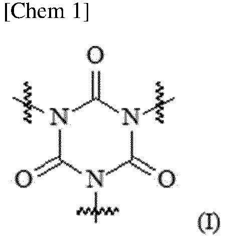

Tel qu'utilisé ici, le terme « groupe (méth)acrylate » signifie indifféremment un groupe acrylate (également appelé acryloyloxy de formule -O-CO-CH=CH₂) ou un groupe méthacrylate (également appelé méthacryloyloxy de formule -O-CO-C(CH₃)=CH₂).

Le mélange selon l'invention peut notamment comprendre un isocyanurate de tris(hydroxyalkyle) (méth)acrylé et un polyester (méth)acrylé à base d'isocyanurate de tris(hydroxyalkyle).

Au sens de l'invention, le terme « isocyanurate de tris(hydroxyalkyle) » ou « THAIC » signifie un composé répondant à la formule (II) suivante : 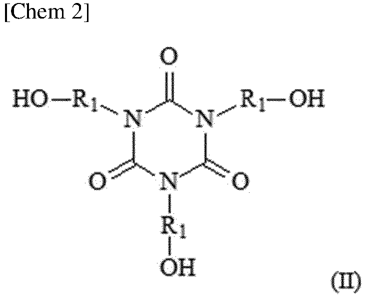 dans laquelle chaque R₁ est indépendamment un alkylène en C2-C12 éventuellement alkoxylé.

En particulier, le THAIC peut correspondre à la formule (II) dans laquelle chaque groupe R₁ est un éthylène (-CH₂-CH₂-). Dans ce cas, le THAIC est un isocyanurate de tris(hydroxyéthyle) ou THEIC.

Au sens de l'invention, le terme « isocyanurate de tris(hydroxyalkyle) (méth)acrylé » ou « THAIC (méth)acrylé » correspond à un isocyanurate de tris(hydroxyalkyle) (THAIC) dans lequel au moins un des groupes OH est transformé en groupe (méth)acrylate (i.e. par estérification avec l'acide (méth)acrylique ou un dérivé d'acide (méth)acrylique). Un THAIC (méth)acrylé peut notamment comprendre un ou plusieurs composés choisis parmi un mono-, di- et tri(méth)acrylate d'isocyanurate de tris(hydroxyalkyl). Ces composés répondent notamment à la formule (III) suivante : 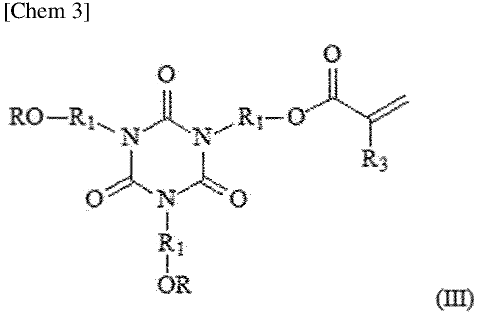 dans laquelle
chaque R est indépendamment H ou un groupe (méth)acryloyl de formule -CO-C(R₃)=CH₂;
chaque R₁ est indépendamment un alkylène en C2-C12 éventuellement alkoxylé ;
chaque R₃ est indépendamment H ou méthyle.

En particulier, le THAIC (méth)acrylé peut correspondre à la formule (III) dans laquelle chaque groupe R₁ est un éthylène (-CH₂-CH₂-). Dans ce cas, le THAIC (méth)acrylé est un isocyanurate de tris(hydroxyéthyle) (méth)acrylé ou THEIC (méth)acrylé.

Un composé de formule (III) dans laquelle chaque groupe R est un groupe (méth)acryloyl de formule -CO-C(R₃)=CH₂ est un tri(méth)acrylate de tris(hydroxyalkyle) isocyanurate ou THAICT(M)A.

Un composé de formule (III) dans laquelle chaque groupe R₁ est un éthylène (-CH₂-CH₂-) et chaque groupe R est un groupe (méth)acryloyl de formule -CO-C(R₃)=CH₂ est un tri(méth)acrylate de tris(hydroxyéthyle) isocyanurate ou THEICT(M)A.

Au sens de l'invention, le terme « polyester » correspond à une molécule polymère comprenant au moins deux liaisons ester. Un polyester peut être constitué d'unités monomères identiques et/ou différentes, de préférence de 2 à 50, et plus préférentiellement de 2 à 10, unités monomères identiques et/ou différentes, obtenues par polycondensation entre au moins un polyacide (ou acide polycarboxylique) et au moins un polyol. Au sens de l'invention, le terme « polyester (méth)acrylé » correspond à un polyester fonctionnalisé par au moins un groupe (méth)acrylate. Au sens de l'invention, le terme « polyester (méth)acrylé à base de tris(hydroxyalkyl) isocyanurate » correspond à un polyester (méth)acrylé incorporant des unités monomères issues d'un THAIC et/ou d'un THAIC (méth)acrylé. En particulier, le polyester (méth)acrylé à base de tris(hydroxyalkyl) isocyanurate peut être un polyester (méth)acrylé à base de tris(hydroxyéthyl) isocyanurate, c'est à dire un polyester (méth)acrylé incorporant des unités monomères issues d'un THEIC et/ou d'un THEIC (méth)acrylé.

Le mélange d'isocyanurates (méth)acrylés selon l'invention est précisément obtenu par réaction entre :
(a) au moins un acide dicarboxylique,
(b) au moins un monomère (méth)acrylique et éventuellement au moins un acide monocarboxylique,
(c) au moins un isocyanurate de tris(hydroxyalkyle) et éventuellement un autre polyol.

Les différents constituants du mélange réactionnel utilisé pour la fabrication du mélange d'isocyanurates selon l'invention seront à présent décrits plus en détail.

### Acide dicarboxylique

Le constituant (a) utilisé dans la fabrication du mélange d'isocyanurates (méth)acrylés selon l'invention comprend au moins un acide dicarboxylique. Le constituant (a) utilisé dans la fabrication du mélange d'isocyanurates (méth)acrylés selon l'invention peut comprendre un mélange d'acides dicarboxyliques.

L'acide dicarboxylique peut notamment être saturé ou insaturé, linéaire, ramifié ou cyclique. L'acide dicarboxylique peut notamment être choisi parmi : les acides dicarboxyliques aliphatiques saturés, les acides dicarboxyliques aliphatiques insaturés, les acides dicarboxyliques cycloaliphatiques saturés, les acides dicarboxyliques cycloaliphatiques insaturés, les acides dicarboxyliques aromatiques et leurs mélanges.

Des exemples d'acides dicarboxyliques aliphatiques saturés sont notamment l'acide adipique, l'acide sébacique, l'acide succinique, l'acide 2-méthylsuccinique, l'acide 2-éthylsuccinique, l'acide 2,2-diméthylsuccinique, l'acide 1,11-undécanedioïque, l'acide 1,12-dodécanedioïque, l'acide oxalique, l'acide malonique, l'acide 2-méthylmalonique, l'acide 2-éthylmalonique, l'acide glutarique, l'acide 3,3-diméthylglutarique, l'acide 3,3-diéthylglutarique, l'acide pimélique, l'acide subérique, l'acide azélaïque ou un dimère d'acide gras en C32-C36.

Des exemples d'acides dicarboxyliques aliphatiques insaturés sont notamment l'acide itaconique, l'acide citraconique, l'acide mésaconique, l'acide glutaconique, l'acide muconique, l'acide fumarique ou l'acide maléique.

Des exemples d'acides dicarboxyliques cycloaliphatiques saturés sont notamment l'acide cyclopentane 1,2- ou 1,3-dicarboxylique, l'acide cyclohexane 1,2-, 1,3- ou 1,4-dicarboxylique, l'acide cycloheptane 1,2- dicarboxylique, le 1,2-, 1,3 ou 1,4-bis(carboxyméthyl)cyclohexane.

Un exemple d'acide dicarboxylique cycloaliphatique insaturé est l'acide tétrahydrophtalique.

Des exemples d'acides dicarboxyliques aromatiques sont l'acide phtalique, l'acide isophtalique, l'acide téréphtalique, le bis(4-carboxyphényl)méthane.

Dans le contexte de cette description, on entend par "acide dicarboxylique" aussi bien les acides dicarboxyliques eux-mêmes que les dérivés d'acides dicarboxyliques. Un tel dérivé peut être transformé en acide dicarboxylique par hydrolyse. Les dérivés d'acide dicarboxylique incluent les formes partiellement ou totalement estérifiées des acides dicarboxyliques définis ci-dessus, notamment les mono- et diesters d'alkyles en C1-C6 des acides dicarboxyliques définis ci-dessus ainsi que les anhydrides cycliques correspondants, les amides correspondants et les halogénures d'acyles correspondants.

Des exemples de dérivés de polyacides de type ester convenables sont le diméthylmalonate, le diéthylmalonate le diméthyladipate, le diméthyl glutarate, le diméthyl succinate.

Le dérivé de polyacide peut notamment être un anhydride cyclique. L'anhydride cyclique peut être saturé ou insaturé, en particulier insaturé. L'anhydride cyclique peut être cycloaliphatique ou aromatique, en particulier aromatique.

Des exemples d'anhydrides cycliques saturés sont l'anhydride succinique et l'anhydride hexahydrophtalique.

Des exemples d'anhydrides insaturés cycloaliphatiques sont l'anhydride maléique, l'anhydride fumarique et l'anhydride tétrahydrophtalique.

Un exemple d'anhydride aromatique est l'anhydride phtalique.

Les dérivés d'acide dicarboxylique sont avantageusement choisis parmi les diesters et anhydrides cycliques de ceux-ci.

Les acides dicarboxylique eux-mêmes, ainsi que leurs dérivés, peuvent être utilisés seuls ou sous forme de mélanges comprenant plusieurs acides dicarboxyliques, plusieurs dérivés d'acides dicarboxyliques ou au moins un acide dicarboxylique et au moins un dérivé d'acide dicarboxylique.

Selon un mode de réalisation préféré, l'acide dicarboxylique est un acide dicarboxylique aliphatique saturé, de préférence un acide dicarboxylique aliphatique saturé en C4-C10, plus préférentiellement un acide dicarboxylique choisi parmi l'acide adipique, l'acide sébacique, l'acide succinique et leurs mélanges, plus préférentiellement encore un mélange d'acide succinique et sébacique.

### Monomère (méth)acrylique

Le constituant (b) utilisé dans la fabrication du mélange d'isocyanurates (méth)acrylés selon l'invention comprend au moins un monomère (méth)acrylique choisi parmi l'acide acrylique, l'acide méthacrylique, leurs anhydrides ou leurs mélanges.

Dans une forme d'exécution de l'invention, le monomère (méth)acrylique est constitué d'un mélange d'acide acrylique et d'acide méthacrylique, de préférence dans un ratio molaire de 5:95 à 95:5, plus préférentiellement de 5:95 à 15:85 ou de 95:5 à 85:15.

On préfère en outre que le ratio molaire du constituant (a) au constituant (b) soit compris entre 1:10 et 1:25, de préférence entre 1:12 et 1:22 et plus préférentiellement entre 1:14 et 1:20.

### Acide monocarboxylique

Le constituant (b) utilisé dans la fabrication du mélange d'isocyanurates (méth)acrylés selon l'invention peut comprendre, outre le monomère (méth)acrylique, au moins un autre acide monocarboxylique en C6-C24 (i.e. un acide monocarboxylique ayant de 6 à 24 atomes de carbone). Dans ce cas, on préfère que le ratio molaire de l'acide monocarboxylique au monomère (méth)acrylique soit compris entre 5:95 et 15:85.

L'acide monocarboxylique peut notamment être saturé ou insaturé, linéaire ou ramifié. L'acide monocarboxylique peut notamment être choisi parmi : les acides monocarboxyliques saturés, les acides monocarboxyliques monoinsaturés, les acides monocarboxyliques polyinsaturés et leurs mélanges.

Des exemples d'acides monocarboxyliques saturés sont notamment les acides hexanoïque, heptanoïque, octanoïque, isooctanoïque, nonanoïque, isononanoïque (ou cékanoïque), décanoïque, undécanoïque, dodécanoïque, tridécanoïque, tétradécanoïque, pentadécanoïque, hexadécanoïque, heptadécanoïque, octadécanoïque, 12-hydroxy-octadécanoïque, nonadécanoïque, eicosanoïque14-hydroxy-eicosanoïque et leurs mélanges. Au sens de l'invention, l'acide isooctanoïque est un acide monocarboxylique ramifiée en C8 (i.e. ayant 8 atomes de carbone) et l'acide isononanoïque est un acide monocarboxylique ramifiée en C9 (i.e. ayant 9 atomes de carbone). Un exemple particulier d'acide isononanoïque est l'acide 3,5,5-triméthylhexanoïque

Des exemples d'acides monocarboxyliques monoinsaturés sont notamment l'acide myristoléique, l'acide palmitoléique, l'acide sapiénique, l'acide oléique, l'acide gadoléique, l'acide ricinoléique (acide 12-hydroxy-9-octadécènoïque), l'acide élaïdique, l'acide trans-vaccénique, l'acide érucique, l'acide nervonique, l'acide brassidique, l'acide lesquérolique (acide 14-hydroxy-11-eicosénoïque) et leurs mélanges.

Des exemples d'acides monocarboxyliques polyinsaturés sont notamment les acides gras de type oméga-3 et oméga-6, notamment l'acide 7,10,13-hexadécatriénoïque, l'acide 9,12,15-octadécatriénoïque, l'acide 6,9,12,15-octadécatrétraénoïque, l'acide 11,14,17-eicosatriénoïque, l'acide 8,11,14,17-eicosatétraénoïque, l'acide 5,8,11,14,17-eicosapentaénoïque, l'acide 6,9,12,15,18-hénéicosapentaénoïque, l'acide 7,10,13,16,19-docosapentaénoïque, l'acide 4,7,10,13,16,19-docosahexaénoïque, l'acide 9,12,15,18,21-tétracosapentaénoïque, l'acide 6,9,12,15,18,21-tétracosahexaénoïque, l'acide 9,12-octadécadiénoïque, l'acide 6,9,12-octadécatriénoïque, l'acide 11,14-eicosadiénoïque, l'acide 8,11,14-eicosatriénoïque, l'acide 5,8,11,14-eicosatétraénoïque, l'acide 13,16-docosadiénoïque, l'acide 7,10,13,16-docosatétraénoïque, l'acide 4,7,10,13,16-docosapentaénoïque, l'acide 9,12,15,18-tétracosatétraénoïque, l'acide 6,9,12,15,18-tétracosapentaénoïque, et leurs mélanges.

Les acides monocarboxyliques peuvent notamment être issus d'huiles végétales.

Selon un mode de réalisation préféré, l'acide monocarboxylique est l'acide isononanoïque.

### Isocyanurate de tris(hydroxyalkyle) - THAIC

Le constituant (c) utilisé dans la fabrication du mélange d'isocyanurates (méth)acrylés selon l'invention comprend au moins un isocyanurate de tris(hydroxyalkyle) (THAIC).

Le THAIC peut notamment être choisi parmi l'isocyanurate de tris(2-hydroxyméthyle), l'isocyanurate de tris(2-hydroxyéthyle), l'isocyanurate de tris(2-hydroxypropyle), l'isocyanurate de tris(2-hydroxyisopropyle), l'isocyanurate de tris(3-hydroxypropyle), l'isocyanurate de tris(2-hydroxybutyle), l'isocyanurate de tris(4-hydroxybutyle), ainsi que les dérivés alkoxylés (notamment éthoxylés et/ou propoxylés) de ceux-ci. De préférence, l'isocyanurate de tris(hydroxyalkyle) est l'isocyanurate de tris(2-hydroxyéthyle) ou THEIC, répondant à la formule (IV) suivante :

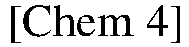

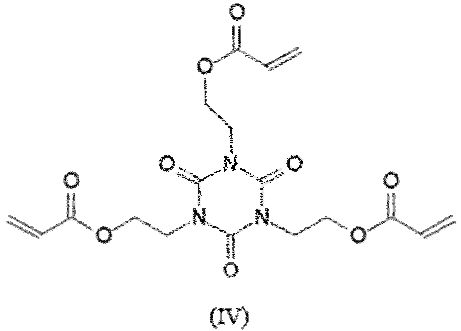

Selon un mode de réalisation préféré, le THAIC représente de 75 à 100%, de préférence 80 à 100%, plus préférentiellement 85 à 100%, molaire du nombre total de moles du constituant (c).

### Autre polyol P_{OH}

Le constituant (c) utilisé dans la fabrication du mélange d'isocyanurates (méth)acrylés selon l'invention peut éventuellement comprendre un polyol autre que THAIC, également désigné P_{OH}.

Dans le cas où P_{OH} est présent, celui-ci peut notamment être choisi parmi les suivants : éthylène glycol, 1,2- ou 1,3-propanediol, 1,2-, 1,3-, 2,3- ou 1,4-butanediol, 1,5-pentanediol, 1,6-hexanediol, 3-méthyl-1,5-pentanediol, 1,10-decanediol, 1,12-dodecanediol, di-, tri- ou polyéthylène glycol, di-, tri ou polypropylène glycol, 1,4-cyclohexanediméthanol, 1,6-cyclohexanediméthanol, 1,4-cyclohexanediol, bisphénol A, bisphénol A hydrogéné, glycérol, diglycérol, tricyclodécane diméthanol, triméthylolpropane, di(triméthylolpropane), triméthyloléthane, 1,2,6-hexanetriol, 1,2,4-butanetriol, érythritol, pentaérythritol, di(pentaérythritol), néopentyl glycol, 2-butyl-2-ethyl-1,3-propanediol, 2-méthyl-1,3-propanediol, 2-méthyl-1,2-propanediol, sorbitol, mannitol, xylitol, isosorbide, isoidide, isomannide, méthyl glucoside, un polyester polyols (notamment polycaprolactone polyol), un polycarbonate polyol, un polyorganosiloxane polyol (notamment polydiméthysiloxane polyol), un polyglycérol (notamment Polyglycérol-3 (trimère de glycérol) et le décaglycérol), un polybutadiène à terminaison hydroxy, un diol dérivé d'un dimère ou trimère d'acide gras hydrogéné ou non, les dérivés alkoxylés (notamment éthoxylés et/ou propoxylés) des polyols cités ci-dessus, et des mélanges de ceux-ci, de préférence le sorbitol.

Selon un mode de réalisation préféré, le mélange réactionnel, en particulier le constituant (c), ne renferme pas d'autre polyol que l'isocyanurate de tris(hydroxyalkyle).

Dans tous les cas, le ratio molaire des groupes -COOH du constituant (a) aux groupes -OH du constituant (c) est compris entre 1:4 et 1:20, de préférence entre 1:5 et 1:15, plus préférablement entre 1:6 et 1:12.

### Procédé de préparation du mélange d'isocyanurates

Le mélange d'isocyanurates (méth)acrylés décrit précédemment peut être obtenu suivant un procédé comprenant les étapes consistant à :
1) faire réagir tout ou partie d'un constituant (b) comprenant au moins un monomère (méth)acrylique choisi parmi l'acide acrylique, l'acide méthacrylique, leurs anhydrides et leurs mélanges et éventuellement au moins un acide monocarboxylique en C6-C24, avec un constituant (c) comprenant au moins un isocyanurate de tris(hydroxyalkyle) et éventuellement un autre polyol P_{OH}, dans des conditions permettant d'estérifier de 50 à 95%, de préférence de 60 à 90%, plus préférentiellement de 70 à 85%, des groupes OH du constituant (c),
2) faire réagir le mélange de l'étape 1) avec un constituant (a) comprenant au moins un acide dicarboxylique, et éventuellement avec le reste, le cas échéant, du constituant (b).

Dans ce procédé, la totalité du monomère (méth)acrylique peut être introduite dans l'étape 1) ou une partie du monomère (méth)acrylique peut être introduite dans l'étape 1) et le reste dans l'étape 2). Dans ce dernier cas de figure, les monomères (méth)acryliques respectivement introduits dans l'étape 1) et l'étape 2) sont avantageusement distincts.

L'étape 1) du procédé selon l'invention est généralement mise en œuvre dans un réacteur équipé d'un système d'agitation. Elle est habituellement réalisée en présence d'un catalyseur d'estérification, d'un inhibiteur de polymérisation, d'un solvant et éventuellement d'un agent de déshydratation. La réaction d'estérification est classiquement favorisée par élimination de l'eau produite lors de la réaction, sous forme de mélange azéotropique avec le solvant. Dans cette étape, les réactifs peuvent être introduits de manière séquentielle ou non. La température est généralement ajustée entre 50 et 120°C et plus préférentiellement entre 80 et 110°C et la réaction peut être conduite ou non sous pression ou sous pression réduite.

Des exemples de solvants utilisables dans l'étape 1) sont les solvants organiques hydrocarbonés, tels que le n-hexane, le n-heptane, le cyclohexane, le méthylcyclohexane, le benzène, le toluène ou le xylène ; les solvants organiques halogénés tels que le dichlorométhane ou le trichlororéthane ; et leurs mélanges. Le solvant est de préférence un solvant organique hydrocarboné. Il peut représenter de 5 à 150% en poids, et de préférence de 50 à 100% en poids, par rapport à la quantité totale de monomère (méth)acrylique et de polyol.

De son côté, le catalyseur d'estérification peut être choisi notamment parmi les acides inorganiques tels que l'acide chlorhydrique, l'acide sulfurique et l'acide phosphorique ; les sels d'acides inorganiques tels que le bisulfate de diammonium, de disodium ou de dipotassium, l'hydrogénophosphate d'ammonium, de sodium ou de potassium, le phosphate d'ammonium, de sodium ou de potassium ; les acides organiques, notamment les acides alkyl- ou arylsulfoniques tels que l'acide para-toluènesulfonique, l'acide 2-naphtalènesulfonique, l'acide méthanesulfonique, l'acide trifluorométhanesulfonique, l'acide éthanesulfonique et l'acide benzènesulfonique ; et leurs mélanges. Le catalyseur est de préférence choisi parmi les acides organiques. Il peut représenter de 1 à 5% et de préférence de 1,5 à 3,5% en poids de la quantité totale de monomère (méth)acrylique et de polyol.

Des exemples d'inhibiteurs de polymérisation sont : les quinones telles que l'hydroquinone, la méthoxyhydroquinone, la para-benzoquinone ; les catéchols tels que le tert-butylcatéchol ; le para-hydroxyanisole ; les mono-, di- et trialkylphénols, tels que le 2-tert-butyl-4-méthylphénol, le 6-tert-butyl-2,4-diméthylphénol, le 2,6-di-tert-butyl-4-méthylphénol, le 2-tert-butylphénol, le 4-tert-butylphénol, le 2,4-di-tert-butylphénol, le 2-méthyl-4-tert-butylphénol, le 4-tert-butyl-2,6-diméthylphénol ; la phénothiazine ; les acides phosphoreux et hypophosphoreux ; les sels de cuivre ou de manganèse, tels que le chlorure de cuivre, l'acétate de cuivre, le sulfate de cuivre, le chlorure de manganèse, l'acétate de manganèse et le sulfate de manganèse ; et leurs mélanges. L'inhibiteur de polymérisation peut représenter de 0,1 à 2,5% et de préférence de 0,5 à 1,5% en poids de la quantité totale de monomère (méth)acrylique et de polyol.

La réaction d'estérification de l'étape 1) est conduite dans des conditions permettant d'estérifier de 50 à 95%, de préférence de 60 à 90%, plus préférentiellement de 70 à 85%, des groupements hydroxyle du constituant (c), tel que mesuré par l'indice d'acide, comme indiqué dans les exemples ci-après.

Le mélange obtenu à l'issue de l'étape 1) du procédé selon l'invention comprend du THAIC (méth)acrylé tel que défini précédemment. Le THAIC (méth)acrylé comprend ainsi un mélange de mono-, di- et/ou tri(méth)acrylates de THAIC, le tri(méth)acrylate de THAIC (aussi désigné par THAICT(M)A) étant majoritaire.

Si le constituant c) utilisé dans l'étape 1) du procédé selon l'invention comprend un autre polyol P_{OH}, le produit obtenu à l'issue de l'étape 1) comprendra en outre du P_{OH} (méth)acrylé, à savoir un mélange de P_{OH} totalement (méth)acrylé (provenant de l'estérification totale de P_{OH} par le monomère (méth)acrylique) et de P_{OH} sous-(méth)acrylé (provenant de l'estérification partielle de P_{OH} par le monomère (méth)acrylique).

Dans l'étape 2) du procédé selon l'invention, l'acide dicarboxylique et éventuellement l'acide monocarboxylique sont mis à réagir avec le produit de l'étape 1), afin d'estérifier les fonctions hydroxyles résiduelles du THAIC (méth)acrylé et éventuellement du P_{OH} (méth)acrylé. Cette étape de polyestérification est généralement conduite à reflux.

Le produit ainsi obtenu peut être isolé par distillation de l'eau. Il est ensuite avantageusement lavé à l'aide d'une solution alcaline aqueuse, avant de séparer la phase organique, notamment par décantation. Celle-ci peut alors être soumise éventuellement à de nouveaux lavages à l'aide d'une solution alcaline aqueuse ou d'eau. Finalement, le solvant est distillé, généralement sous pression réduite.

Le mélange obtenu avec le procédé selon l'invention peut notamment comprendre:
- du THAICT(M)A ;
- éventuellement du P_{OH} totalement (méth)acrylé ; et
- un composant polyester PE.

Le composant polyester PE peut notamment comprendre des polyesters à base d'acide dicarboxylique et de mono- et/ou di(méth)acrylates de THAIC et éventuellement de P_{OH} sous-(méth)acrylé. Le composant polyester PE peut notamment comprendre ou consister en un mélange de composés répondant à la formule (V) suivante : 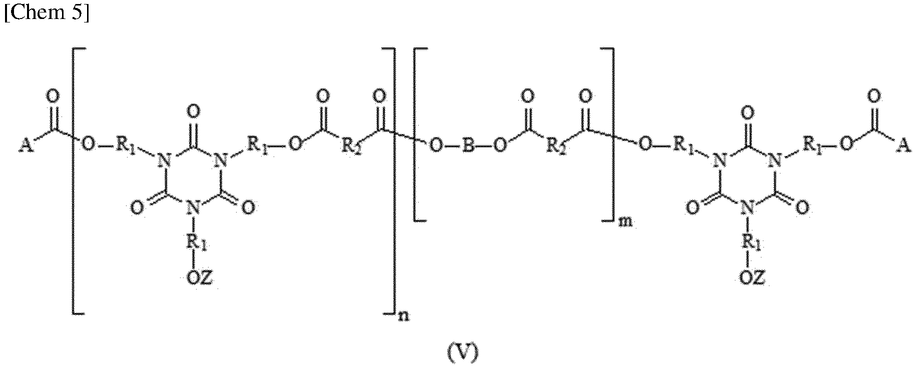 dans laquelle
chaque R₁ est indépendamment un alkylène en C2-C12 éventuellement alkoxylé ;
chaque R₂ est indépendamment le résidu d'un acide dicarboxylique ;
chaque A est indépendamment un résidu d'acide (méth)acrylique ou d'acide monocarboxylique en C6-C24, de préférence un résidu d'acide (méth)acrylique ;
chaque B est indépendamment le résidu d'un polyol P_{OH} autre que THAIC ;
chaque Z est indépendamment H ou -C(=O)-A ;
m et n sont des valeurs moyennes où n va de 1 à 10, de préférence n va de 1 à 2, et m va de 0 à 10, de préférence m est égal à 0.

Le composant polyester PE comprend de préférence au moins un composé de formule (V) dans laquelle au moins un des groupes A, de préférence chaque groupe A, correspond à un résidu d'acide (méth)acrylique, c'est-à-dire un groupe de formule -C(R₃)=CH₂ dans lequel R₃ est H ou méthyle. Le composant polyester PE peut notamment comprendre au moins un composé de formule (V) dans laquelle au moins un des groupes Z correspond à un groupe -C(=O)-C(R₃)=CH₂. Le composant polyester PE peut notamment comprendre au moins un composé de formule (V) dans laquelle au moins un des groupes Z correspond à un groupe H.

Plus préférentiellement, le composant polyester PE comprend ou consiste en un mélange de composés répondant à la formule (VI) suivante : 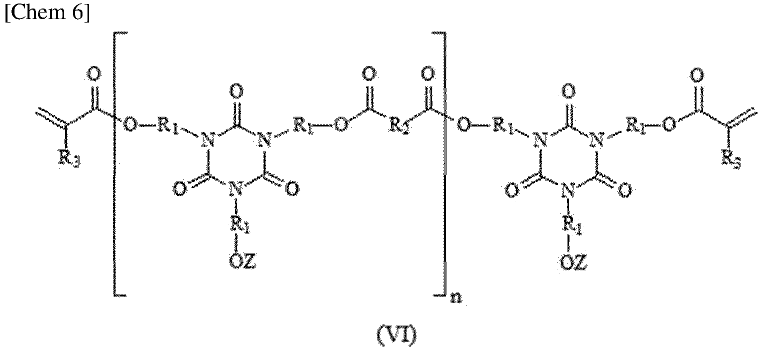 dans laquelle
chaque R₁ est indépendamment un alkylène en C2-C12 éventuellement alkoxylé ;
chaque R₂ est indépendamment le résidu d'un acide dicarboxylique ;
chaque R₃ est indépendamment H ou méthyle ;
chaque Z est indépendamment H ou -C(=O)-C(R₃)=CH₂ ;
n va de 1 à 10, de préférence n va de 1 à 2.

Le composant polyester PE peut notamment comprendre au moins un composé de formule (VI) dans laquelle au moins un des groupes Z correspond à un groupe -C(=O)-C(R₃)=CH₂. Le composant polyester PE peut notamment comprendre au moins un composé de formule (VI) dans laquelle au moins un des groupes Z correspond à un groupe H.

Le mélange selon l'invention peut notamment comprendre 40 à 90%, de préférence 45 à 85%, plus préférentiellement 50 à 80%, en poids de THAICT(M)A par rapport au poids du mélange (hors solvant éventuel).

Le mélange selon l'invention peut notamment comprendre 10 à 60%, de préférence 15 à 55%, plus préférentiellement 20 à 50% en poids de composant polyester PE par rapport au poids du mélange (hors solvant éventuel).

### Composition polymérisable

Un autre objet de la présente invention concerne une composition polymérisable comprenant au moins un mélange d'isocyanurates (méth)acrylés tel que défini selon la présente invention et éventuellement au moins un autre composé éthyléniquement insaturé.

Au sens de l'invention, un « composé éthyléniquement insaturé » signifie un composé qui comprend une double liaison carbone-carbone polymérisable. Une double liaison carbone-carbone polymérisable est une double liaison carbone-carbone qui peut réagir avec une autre double liaison carbone-carbone dans une réaction de polymérisation. Une double liaison carbone-carbone polymérisable est généralement comprise dans un groupe choisi parmi acrylate (y compris cyanoacrylate), méthacrylate, acrylamide, méthacrylamide, styrène, maléate, fumarate, itaconate, allyle, propényle, vinyle et des combinaisons correspondantes, préférablement choisi parmi acrylate, méthacrylate et vinyle, plus préférablement choisi parmi acrylate et méthacrylate. Les doubles liaisons carbone-carbone d'un cycle phényle ne sont pas considérées comme des doubles liaisons carbone-carbone polymérisables.

Dans un mode de réalisation, le composé éthyléniquement insaturé peut être choisi parmi un monomère fonctionnalisé par (méth)acrylate, un oligomère fonctionnalisé par (méth)acrylate et des mélanges correspondants. En particulier, le composé éthyléniquement insaturé comprend un monomère fonctionnalisé par (méth)acrylate.

La quantité totale de composé éthyléniquement insaturé dans la composition polymérisable peut être de 0 à 90 %, en particulier 5 à 85 %, plus particulièrement 10 à 80 %, en poids sur la base du poids de la composition. En particulier, la composition polymérisable peut comprendre 0 à 60 %, ou 5 à 60 % ou 10 à 60 % ou 15 à 60 % ou 20 à 60 % en poids de composé éthyléniquement insaturé sur la base du poids de la composition. En variante, la composition polymérisable peut comprendre 50 à 80 %, ou 55 à 80 % ou 60 à 80 %, en poids de composé éthyléniquement insaturé sur la base du poids de la composition.

Tel qu'utilisé ici, le terme « monomère fonctionnalisé par (méth)acrylate » signifie un monomère comprenant au moins un groupe (méth)acryloyloxy, en particulier un groupe acryloyloxy. Le terme « oligomère fonctionnalisé par (méth)acrylate » signifie un oligomère comprenant un groupe (méth)acryloyloxy, en particulier un groupe acryloyloxy.

Dans un mode de réalisation, le composé éthyléniquement insaturé comprend un monomère fonctionnalisé par (méth)acrylate. Le composé éthyléniquement insaturé peut comprendre un mélange de monomères fonctionnalisés par (méth)acrylate.

Le monomère fonctionnalisé par (méth)acrylate peut présenter un poids moléculaire inférieur à 600 g/mol, en particulier de 100 à 550 g/mol, plus particulièrement de 200 à 500 g/mol.

Le monomère fonctionnalisé par (méth)acrylate peut avoir 1 à 6 groupes (méth)acryloyloxy, en particulier 1 à 4 groupes (méth)acryloyloxy.

Le monomère fonctionnalisé par (méth)acrylate peut comprendre un mélange de monomères fonctionnalisés par (méth)acrylate ayant différentes fonctionnalités. Par exemple, le monomère fonctionnalisé par (méth)acrylate peut comprendre un mélange d'un monomère fonctionnalisé par (méth)acrylate contenant un unique groupe acryloyloxy ou méthacryloyloxy par molécule (dénommé ici « composés fonctionnalisés par mono(méth)acrylate ») et d'un monomère fonctionnalisé par (méth)acrylate contenant 2 ou plus, préférablement 2 ou 3, groupes acryloyloxy et/ou méthacryloyloxy par molécule.

Dans un mode de réalisation, le monomère fonctionnalisé par (méth)acrylate comprend un monomère fonctionnalisé par mono(méth)acrylate. Le monomère fonctionnalisé par mono(méth)acrylate peut avantageusement fonctionner comme un diluant réactif et réduire la viscosité de la composition polymérisable de l'invention.

Des exemples de monomères fonctionnalisés par mono(méth)acrylate appropriés comprennent, mais ne sont pas limités à, des esters de mono(méth)acrylate d'alcools aliphatiques (l'alcool aliphatique pouvant être à chaîne droite, ramifié ou alicyclique et pouvant être un monoalcool, un dialcool ou un polyalcool, à condition qu'un seul groupe hydroxyle soit estérifié par un acide (méth)acrylique) ; des esters de mono(méth)acrylate d'alcools aromatiques (tels que des phénols, y compris des phénols alkylés) ; des esters de mono(méth)acrylate d'alcools d'alkylaryle (tels que l'alcool benzylique) ; des esters de mono(méth)acrylate de glycols oligomériques et polymériques tels que le diéthylèneglycol, le triéthylèneglycol, le dipropylèneglycol, le tripropylèneglycol, un polyéthylèneglycol, et un polypropylèneglycol) ; des esters de mono(méth)acrylate d'éthers de monoalkyle de glycols et d'oligoglycols ; des esters de mono(méth)acrylate d'alcools aliphatiques alcoxylés (par ex., éthoxylés et/ou propoxylés) (l'alcool aliphatique pouvant être à chaîne droite, ramifié ou alicyclique et pouvant être un monoalcool, un dialcool ou un polyalcool, à condition qu'un seul groupe hydroxyle de l'alcool aliphatique alcoxylé soit estérifié avec un acide (méth)acrylique) ; des esters de mono(méth)acrylate d'alcools aromatiques (tels que des phénols alcoxylés) alcoxylés (par ex., éthoxylés et/ou propoxylés) ; des mono(méth)acrylates de caprolactone ; et similaire.

Les composés suivants sont des exemples spécifiques de monomères fonctionnalisés par mono(méth)acrylate appropriés pour une utilisation dans les compositions polymérisables de la présente invention : (méth)acrylate de méthyle ; (méth)acrylate d'éthyle ; (méth)acrylate de n-propyle ; (méth)acrylate de n-butyle ; (méth)acrylate d'isobutyle ; (méth)acrylate de n-hexyle ; (méth)acrylate de 2-éthylhexyle ; (méth)acrylate de n-octyle ; (méth)acrylate d'isooctyle ; (méth)acrylate de n-décyle ; (méth)acrylate de n-dodécyle ; (méth)acrylate de tridécyle ; (méth)acrylate de tétradécyle ; (méth)acrylate d'hexadécyle ; (méth)acrylate de 2-hydroxyéthyle ; (méth)acrylate de 2-hydroxypropyle et (méth)acrylate de 3-hydroxypropyle ; (méth)acrylate de 2-méthoxyéthyle ; (méth)acrylate de 2-éthoxyéthyle ; (méth)acrylate de 2-éthoxypropyle et (méth)acrylate de 3-éthoxypropyle ; (méth)acrylate de tétrahydrofurfuryle ; (méth)acrylate de tétrahydrofurfuryle alcoxylé ; (méth)acrylate de 2-(2-éthoxyéthoxy)éthyle ; (méth)acrylate de cyclohexyle ; (méth)acrylate de glycidyle ; (méth)acrylate d'isodécyle ; (méth)acrylate de lauryle ; (méth)acrylate de 2-phénoxyéthyle ; (méth)acrylates de phénol alcoxylé ; (méth)acrylates de nonylphénol alcoxylé ; (méth)acrylate de triméthylolpropane formal cyclique ; (méth)acrylate d'isobornyle ; (méth)acrylate de tricyclodécaneméthanol ; (méth)acrylate de tert-butylcyclohexanol ; (méth)acrylate de triméthylcyclohexanol ; (méth)acrylate de monométhyléther de diéthylèneglycol ; (méth)acrylate de monobutyléther de diéthylèneglycol ; (méth)acrylate de monoéthyléther de triéthylèneglycol ; (méth)acrylate de lauryle éthoxylé ; (méth)acrylates de méthoxy polyéthylèneglycol ; (méth)acrylates d'hydroxyle éthyl-butyle uréthane ; (méth)acrylates de 3-(2-hydroxyalkyle)oxazolidinone ; et des combinaisons correspondantes.

Dans un mode de réalisation, le monomère fonctionnalisé par (méth)acrylate peut comprendre un monomère fonctionnalisé par (méth)acrylate contenant deux groupes (méth)acryloyloxy ou plus par molécule.

Des exemples de monomères fonctionnalisés par (méth)acrylate appropriés contenant deux groupes de type (méth)acryloyloxy ou plus par molécule comprennent des esters d'acrylate et de méthacrylate de polyols (des composés organiques contenant deux groupes hydroxyle ou plus par molécule, par ex. 2 à 6). Des exemples spécifiques de polyols appropriés sont tels que définis précédemment pour P et P'. De tels polyols peuvent être estérifiés totalement ou partiellement (avec un acide (méth)acrylique, un anhydride (méth)acrylique, un chlorure de (méth)acryloyle ou similaire), à condition qu'ils contiennent au moins deux groupes fonctionnels de type (méth)acryloyloxy par molécule.

Des exemples de monomères fonctionnalisés par (méth)acrylate contenant deux groupes (méth)acryloyloxy ou plus par molécule peuvent comprendre des di(méth)acrylate de bisphénol A; di(méth)acrylate de bisphénol A hydrogéné; di(méth)acrylate d'éthylène glycol; di(méth)acrylate de diéthylène glycol; di(méth)acrylate de triéthylène glycol; di(méth)acrylate de tétraéthylène glycol; di(méth)acrylate de polyéthylène glycol; di(méth)acrylate de propylène glycol; di(méth)acrylate de dipropylène glycol; di(méth)acrylate de tripropylène glycol; di(méth)acrylate tétrapropylène glycol; di(méth)acrylate de polypropylène glycol; di(méth)acrylate de polytétraméthylène glycol; di(méth)acrylate de 1,2-butanediol; di(méth)acrylate de 2,3-butanediol; di(méth)acrylate de 1,3-butanediol; di(méth)acrylate de 1,4-butanediol; di(méth)acrylate de 1,5-pentanediol; di(méth)acrylate de 1,6-hexanediol; di(méth)acrylate de 1,8-octanediol; di(méth)acrylate de 1,9-nonanediol; di(méth)acrylate de 1,10-dénanediol di(méth)acrylate; di(méth)acrylate de 1,12-dodécanediol; di(méth)acrylate de néopentyl glycol; di(méth)acrylate de 2-méthyl-2,4-pentanediol; di(méth)acrylate de polybutadiène; di(méth)acrylate de cyclohexane-1,4-diméthanol; di(méth)acrylate de tricyclodécane diméthanol; des di(méth)acrylates métalliques; des di(méth)acrylates métalliques modifiés; di(méth)acrylate de glycérol; tri(méth)acrylate de glycérol; tri(méth)acrylate de triméthyloléthane; di(méth)acrylate de triméthyloléthane; tri(méth)acrylate de triméthylolpropane; di(méth)acrylate de triméthylolpropane; di(méth)acrylate de pentaérythritol; tri(méth)acrylate de pentaérythritol; tétra(méth)acrylate de pentaérythritol, di(méth)acrylate de di(triméthylolpropane); tri(méth)acrylate de di(triméthylolpropane); tétra(méth)acrylate de di(triméthylolpropane), penta(méth)acrylate de sorbitol; tétra(méth)acrylate de di(pentaérythritol); penta(méth)acrylate de di(pentaérythritol); hexa(méth)acrylate de di(pentaérythritol); tri(méth)acrylate de tris(2-hydroxyethyl) isocyanurate; ainsi que les dérivés alcoxylés (e.g., ethoxylés and/or propoxylés) de ceux-ci; et les mélanges de ceux-ci.

La composition polymérisable de l'invention peut comprendre 0 à 90 %, en particulier 5 à 85 %, plus particulièrement 10 à 80 %, en poids de monomère fonctionnalisé par (méth)acrylate sur la base du poids de la composition. En particulier, la composition polymérisable peut comprendre 0 à 60 %, ou 5 à 60 % ou 10 à 60 % ou 15 à 60 % ou 20 à 60 % en poids de monomère fonctionnalisé par (méth)acrylate sur la base du poids de la composition. En variante, la composition polymérisable peut comprendre 50 à 80%, ou 55 à 80%, ou 60 à 80%, en poids de monomère fonctionnalisé par (méth)acrylate sur la base du poids de la composition.

Dans un mode de réalisation, le composé éthyléniquement insaturé comprend un oligomère fonctionnalisé par (méth)acrylate. Le composé éthyléniquement insaturé peut comprendre un mélange d'oligomères fonctionnalisés par (méth)acrylate.

L'oligomère fonctionnalisé par (méth)acrylate peut être choisi afin d'augmenter la flexibilité, la résistance et/ou le module, parmi d'autres attributs, d'un polymère durci préparé à l'aide de la composition polymérisable de la présente invention.

L'oligomère fonctionnalisé par (méth)acrylate peut avoir 1 à 18 groupes (méth)acryloyloxy, en particulier 2 à 6 groupes (méth)acryloyloxy, plus particulièrement 2 à 6 groupes acryloyloxy.

L'oligomère fonctionnalisé par (méth)acrylate peut présenter un poids moléculaire moyen en nombre supérieur ou égal à 600 g/mol, en particulier 800 à 15 000 g/mol, plus particulièrement 1 000 à 5 000 g/mol.

En particulier, les oligomères fonctionnalisés par (méth)acrylate peuvent être choisis dans le groupe consistant en des oligomères d'uréthane fonctionnalisés par (méth)acrylate (parfois également dénommés « oligomères de (méth)acrylate d'uréthane » « oligomères de (méth)acrylate de polyuréthane » ou « oligomères de (méth)acrylate de carbamate »), des oligomères d'époxy fonctionnalisés par (méth)acrylate (parfois également dénommés « oligomères de (méth)acrylate d'époxy »), des oligomères de polyéther fonctionnalisés par (méth)acrylate (parfois également dénommés « oligomères de (méth)acrylate de polyéther »), des oligomères de polydiène fonctionnalisés par (méth)acrylate (parfois également dénommés « oligomères de (méth)acrylate de polydiène »), des oligomères de polycarbonate fonctionnalisés par (méth)acrylate (parfois également dénommés « oligomères de (méth)acrylate de polycarbonate »), et des oligomères de polyester fonctionnalisés par (méth)acrylate (parfois également dénommés « oligomères de (méth)acrylate de polyester ») autres que celui selon l'invention et des mélanges correspondants.

Des oligomères de (méth)acrylate de polyester donnés à titre d'exemple comprennent les produits de réaction d'acide acrylique ou méthacrylique ou des mélanges ou des équivalents synthétiques correspondants avec des polyester polyols terminés par un groupe hydroxyle. Le procédé de réaction peut être conduit de sorte que tous, ou essentiellement tous, les groupes hydroxyle du polyester polyol ont été (méth)acrylés, particulièrement dans des cas où le polyester polyol est difonctionnel. Les polyester polyols peuvent être préparés par des réactions de polycondensation de composants fonctionnalisés par polyhydroxyle (en particulier, des diols) et des composés fonctionnalisés par poly(acide carboxylique) (en particulier, des acides et des anhydrides dicarboxyliques). Les composants fonctionnalisés par polyhydroxyle et fonctionnalisés par poly(acide carboxylique) peuvent chacun avoir des structures linéaires, ramifiées, cycloaliphatiques ou aromatiques et peuvent être utilisés individuellement ou en tant que mélanges.

Des exemples de (méth)acrylates d'époxy appropriés comprennent les produits de réaction d'acide acrylique ou méthacrylique ou des mélanges correspondants avec une résine époxy (éther ou ester de polyglycidyle). La résine époxy peut, en particulier, être choisie parmi l'éther de diglycidyle de bisphénol A, l'éther de diglycidyle de bisphénol F, l'éther de diglycidyle de bisphénol S, l'éther de diglycidyle de bisphénol A bromé, l'éther de diglycidyle de bisphénol F bromé, l'éther de diglycidyle de bisphénol S bromé, une résine novolak époxy, l'éther de diglycidyle de bisphénol A hydrogéné, l'éther de diglycidyle de bisphénol F hydrogéné, l'éther de diglycidyle de bisphénol S hydrogéné, le 3,4-époxycyclohexylméthyl-3',4'-époxycyclohexanecarboxylate, le 2-(3,4-époxycyclohexyl-5,5-spiro-3,4-époxy)cyclohexane-1,4-dioxanne, le bis(3,4-époxycyclohexylméthyl)adipate, l'oxyde de vinylcyclohexène, le 4-vinylépoxycyclohexane, le bis(3,4-époxy-6-méthylcyclohexylméthyl)adipate, le 3,4-époxy-6-méthylcyclohexyl-3',4'-époxy-6'-méthylcyclohexanecarboxylate, le méthylènebis(3,4-époxycyclohexane), le diépoxyde de dicyclopentadiène, le di(3,4-époxycyclohexylméthyl)éther d'éthylèneglycol, l'éthylènebis(3,4-époxycyclohexanecarboxylate), l'éther de diglycidyle de 1,4-butanediol, l'éther de diglycidyle de 1,6-hexanediol, l'éther de triglycidyle de glycérol, l'éther de triglycidyle de triméthylolpropane, l'éther de diglycidyle de polyéthylèneglycol, l'éther de diglycidyle de polypropylèneglycol, des éthers de polyglycidyle d'un polyéther polyol obtenu par l'addition d'un ou plusieurs oxydes d'alkylène à un alcool polyhydrique aliphatique, tel que l'éthylèneglycol, le propylèneglycol, et le glycérol, des esters de diglycidyle d'acides dibasiques aliphatiques à chaîne longue, des éthers de monoglycidyle d'alcools supérieurs aliphatiques, des éthers de monoglycidyle de phénol, de crésol, de butylphénol, ou des polyéther alcools obtenus par l'addition d'oxyde d'alkylène à ces composés, des esters de glycidyle d'acides gras supérieurs, l'huile de soja époxydée, l'acide époxybutylstéarique, l'acide époxyoctylstéarique, l'huile de graines de lin époxydée, un polybutadiène époxydé, et similaire.

Les oligomères de (méth)acrylate de polyéther appropriés comprennent, mais sans s'y limiter, les produits de réaction de condensation d'acide acrylique ou méthacrylique ou des mélanges ou des équivalents synthétiques correspondants avec des polyétherols qui sont des polyéther polyols (tels qu'un polyéthylène glycol, un polypropylène glycol ou un polytétraméthylène glycol). Les polyétherols appropriés peuvent être des substances linéaires ou ramifiées contenant des liaisons éther et des groupes hydroxyle terminaux. Les polyétherols peuvent être préparés par polymérisation par ouverture de cycle d'éthers cycliques tels que le tétrahydrofuranne ou des oxydes d'alkylène (par ex. l'oxyde d'éthylène et/ou l'oxyde de propylène) avec une molécule de départ. Les molécules de départ appropriées comprennent l'eau, des matières fonctionnalisées par polyhydroxyle, des polyester polyols et des amines.

Des oligomères de (méth)acrylate de polyuréthane (parfois également dénommés « oligomères de (méth)acrylate d'uréthane ») appropriés pour une utilisation dans les compositions polymérisables de la présente invention comprennent des uréthanes à base de polyester polyols et polyéther polyols aliphatiques, cycloaliphatiques et/ou aromatiques et des diisocyanates de polyester aliphatiques, cycloaliphatiques et/ou aromatiques et des diisocyanates de polyéther coiffés avec des groupes terminaux (méth)acrylate. Les oligomères de (méth)acrylate de polyuréthane appropriés comprennent, par exemple, des oligomères de diacrylate et tétraacrylate d'uréthane à base de polyester aliphatique, des oligomères de diacrylate et tétraacrylate d'uréthane à base de polyéther aliphatique, ainsi que des oligomères de diacrylate et tétraacrylate d'uréthane à base de polyester/polyéther aliphatique.

Les oligomères de (méth)acrylate de polyuréthane peuvent être préparés par la mise en réaction de polyisocyanates aliphatiques, cycloaliphatiques ou aromatiques (par ex. diisocyanate, triisocyanate) avec des polyester polyols, des polyéther polyols, des polycarbonate polyols, des polycaprolactone polyols, des polyorganosiloxane polyols (par ex. des polydiméthylsiloxane polyols), ou des polydiène polyols (par ex. des polybutadiène polyols), terminés par un groupe OH, ou des combinaisons correspondantes, pour former des oligomères fonctionnalisés par isocyanate qui sont ensuite mis à réagir avec des (méth)acrylates fonctionnalisés par hydroxyle tels que l'acrylate d'hydroxyéthyle ou le méthacrylate d'hydroxyéthyle pour fournir des groupes (méth)acrylate terminaux. Par exemple, les oligomères de (méth)acrylate de polyuréthane peuvent contenir deux, trois, quatre groupes fonctionnels (méth)acrylate par molécule ou plus. D'autres ordres d'addition peuvent également être pratiqués pour préparer le (méth)acrylate de polyuréthane, comme il est connu dans l'état de l'art. Par exemple, le (méth)acrylate fonctionnalisé par hydroxyle peut être d'abord mis à réagir avec un polyisocyanate pour obtenir un (méth)acrylate fonctionnalisé par isocyanate, qui peut ensuite être mis à réagir avec un polyester polyol, un polyéther polyol, un polycarbonate polyol, un polycaprolactone polyol, un polydiméthylsiloxane polyol ou un polybutadiène polyol, terminé par un groupe OH, ou une combinaison correspondante. Dans encore un autre mode de réalisation, un polyisocyanate peut d'abord être mis à réagir avec un polyol, y compris l'un quelconque parmi les types de polyols mentionnés précédemment, pour obtenir un polyol fonctionnalisé par isocyanate, qui est ensuite mis à réagir avec un (méth)acrylate fonctionnalisé par hydroxyle pour donner un (méth)acrylate de polyuréthane. En variante, tous les composants peuvent être combinés et mis à réagir en même temps.

La composition polymérisable de l'invention peut comprendre 0 à 90%, en particulier 5 à 85%, plus particulièrement 10 à 80%, en poids d'oligomère fonctionnalisé par (méth)acrylate sur la base du poids de la composition. En particulier, la composition polymérisable peut comprendre 0 à 60%, ou 5 à 60%, ou 10 à 60%, ou 15 à 60%, ou 20 à 60%, en poids d'oligomère fonctionnalisé par (méth)acrylate sur la base du poids de la composition. En variante, la composition polymérisable peut comprendre 50 à 80%, ou 55 à 80%, ou 60 à 80%, en poids d'oligomère fonctionnalisé par (méth)acrylate sur la base du poids de la composition.

La composition polymérisable de l'invention peut également avantageusement comprendre un amorceur de polymérisation radicalaire ou ionique, et plus particulièrement un photoinitiateur ou un peroxyde.

Le photoinitiateur peut être un photoinitiateur de radicaux, en particulier un photoinitiateur de radicaux ayant une activité de type Norrish I et/ou une activité de type Norrish II, plus particulièrement un photoinitiateur de radicaux ayant une activité de type Norrish I.

Des types non limitants de photoinitiateurs de radicaux appropriés pour une utilisation dans les compositions polymérisables de la présente invention comprennent, par exemple, les benzoïnes, les éthers de benzoïne, les acétophénones, les α-hydroxyacétophénones, le benzile, les cétals de benzile, les anthraquinones, les oxydes de phosphine, les oxydes d'acylphosphine, les α-hydroxycétones, les phénylglyoxylates, les α-aminocétones, les benzophénones, les thioxanthones, les xanthones, les dérivés d'acridine, les dérivés de phénazène, les dérivés de quinoxaline, les composés de triazine, les formiates de benzoyle, les oximes aromatiques, les métallocènes, les composés de type acylsilyle ou acylgermanyle, les camphoquinones, des dérivés polymériques correspondants, et des mélanges correspondants.

Des exemples de photoinitiateurs de radicaux appropriés comprennent, sans s'y limiter, la 2-méthylanthraquinone, la 2-éthylanthraquinone, la 2-chloroanthraquinone, la 2-benzyanthraquinone, la 2-t-butylanthraquinone, la 1,2-benzo-9,10-anthraquinone, le benzile, les benzoïnes, les éthers de benzoïne, la benzoïne, l'éther méthylique de benzoïne, l'éther éthylique de benzoïne, l'éther isopropylique de benzoïne, l'alpha-méthylbenzoïne, l'alpha-phénylbenzoïne, la cétone de Michler, les acétophénones telles que les 2,2-dialcoxybenzophénones et les 1-hydroxyphénylcétones, la benzophénone, la 4,4'-bis-(diéthylamino)benzophénone, l'acétophénone, la 2,2-diéthyloxyacétophénone, la diéthyloxyacétophénone, la 2-isopropylthioxanthone, la thioxanthone, la diéthylthioxanthone, la 1,5-acétonaphtylène, la cétone de benzil, α-hydroxy céto, l'oxyde de 2,4,6-triméthylbenzoyldiphényl phosphine, le diméthylcétal de benzile, la 2,2-diméthoxy-1,2-diphényléthanone, la 1-hydroxycylclohexylphénylcétone, la 2-méthyl-1-[4-(méthylthio) phényl]-2-morpholinopropanone-1, la 2-hydroxy-2-méthyl-1-phényl-propanone, une α-hydroxycétone oligomérique, des oxydes de benzoylphosphine, l'oxyde de phénylbis(2,4,6-triméthylbenzoyl)phosphine, l'éthyl(2,4,6-triméthylbenzoyl)phényl phosphinate, l'anisoïne, l'anthraquinone, le monohydrate du sel de sodium de l'acide anthraquinone-2-sulfonique, le (benzène)tricarbonylchrome, le benzile, l'éther d'isobutyle de benzoïne, un mélange 50/50 benzophénone/1-hydroxycyclohexyl phényl cétone, le dianhydride 3,3',4,4'-benzophénonetétracarboxylique, le 4-benzoylbiphényle, la 2-benzyl-2-(diméthylamino)-4'-morpholinobutyrophénone, la 4,4'-bis(diéthylamino)benzophénone, la 4,4'-bis(diméthylamino)benzophénone, la camphoquinone, la 2-chlorothioxanthén-9-one, la dibenzosubérènone, la 4,4'-dihydroxybenzophénone, la 2,2-diméthoxy-2-phénylacétophénone, la 4-(diméthylamino)benzophénone, le 4,4'-diméthylbenzile, la 2,5-diméthylbenzophénone, la 3,4-diméthylbenzophénone, un mélange 50/50 oxyde de diphényl(2,4,6-triméthylbenzoyl)phosphine/2-hydroxy-2-méthylpropiophénone, la 4'-éthoxyacétophénone, l'oxyde de 2,4,6-triméthylbenzoyldiphénylphophine, l'oxyde de phényl-bis(2,4,6-triméthylbenzoyl)phosphine, le ferrocène, la 3'-hydroxyacétophénone, la 4'-hydroxyacétophénone, la 3-hydroxybenzophénone, la 4-hydroxybenzophénone, la 1-hydroxycyclohexyl phényl cétone, la 2-hydroxy-2-méthylpropiophénone, la 2-méthylbenzophénone, la 3-méthylbenzophénone, le méthybenzoylformiate, la 2-méthyl-4'-(méthylthio)-2-morpholinopropiophénone, la phénanthrènequinone, la 4'-phénoxyacétophénone, l'hexafluorophosphate de (cumène)cyclopentadiényl fer(ii), le 9,10-diéthoxyanthracène et le 9,10-dibutoxyanthracène, le 2-éthyl-9,10-diméthoxyanthracène, la thioxanthén-9-one et des combinaisons correspondantes.

En particulier, le photoinitiateur peut être une benzophénone (telle que SpeedCure^{®} BP, SpeedCure^{®} 7005, SpeedCure^{®} 7006), une thioxanthone (telle que SpeedCure^{®} 7010, SpeedCure^{®} ITX), une α-hydroxyacétophénone (telle que SpeedCure^{®} 73), un oxyde d'acylphosphine (tel que SpeedCure^{®} BPO, SpeedCure^{®} TPO, SpeedCure^{®} TPO-L). Préférablement, le photoinitiateur est une α-hydroxyacétophénone ou un oxyde d'acylphosphine.

La composition polymérisable de l'invention peut notamment comprendre 0 à 20%, en particulier 0,1 à 15%, plus particulièrement 1 à 10% en poids de photoinitiateur par rapport au poids de la composition.

En outre, la composition polymérisable de l'invention peut comprendre un ou plusieurs additifs choisis parmi les : antioxydants, photostabilisants, absorbeurs de lumière, inhibiteurs de polymérisation, agents antimousse, agents antistatiques, agents nivelants, dispersants (agents mouillants, tensioactifs), agents de glissement, promoteurs d'adhérence, lubrifiants, pigments, colorants, charges, agents de transfert de chaine, agents rhéologiques (thixotropique, épaississant), agents matifiants, agents opacifiants, agents de résistance à l'impact, cires.

De préférence, la composition polymérisable de l'invention est une composition d'encre, de revêtement (notamment de revêtement de protection, de revêtement d'isolation électrique, de revêtement décoratif ou de revêtement réactif aux stimulus extérieurs), de matériau chargé avec des renforts fibreux ou particulaires pouvant être des nanotubes de carbone ou du graphite (notamment de mastic, de cheville chimique, de pierre artificielle, de matériau dentaire ou de composite), une composition d'adhésif, de moulage, de plaque encreuse, de liant pour électrode, ou une composition pour la fabrication additive, en particulier pour l'impression d'objets 3D ou 4D.

Au sens de l'invention, une plaque encreuse est une plaque photopolymère souple destinée au transfert de l'encre vers le support à imprimer dans l'impression typographique rotative ou dans la flexographie.

La fabrication additive, appelé aussi impression 3D, consiste à créer, à partir d'un modèle numérique contenant les propriétés liées à la géométrie de l'objet à produire (maillage de points ou de surfaces) et éventuellement les paramètres des matériaux à utiliser, un objet (volumétrique / tridimensionnel) point par point (appelés voxels par analogie avec les pixels de l'impression classique bidimensionnelle), soit en modifiant sélectivement en ces points les propriétés d'un milieu meuble par exemple par solidification (polymérisation) à partir d'un bac de résine liquide, par agglomération/frittage/fusion-resolidification à partir d'un lit de poudre, soit à déposer sélectivement en différents points d'une surface (appelée aussi couche et généralement plane) le matériau en continu (par extrusion) ou discontinu (jet d'encre), et ce, surface après surface. Les surfaces pouvant s'additionner l'une sous l'autre ou l'une sur l'autre, ainsi que du centre vers l'extérieur, généralement à partir d'un support d'impression, le matériau non modifié pouvant éventuellement être lui-même support. Les principes généraux de l'impression 3D sont définis dans la norme ISO/ASTM 52900:2015. L'impression d'un objet 4D peut se définir comme de l'impression d'un objet 3D qui est capable de se transformer au fil du temps. Ainsi, l'impression 4D est le processus par lequel un objet imprimé en 3D peut modifier lui-même sa structure et changer de forme sous l'impulsion d'une énergie extérieure comme la température, la lumière ou d'autres stimulus environnementaux.

La composition polymérisable définie ci-dessus peut être réticulée, en particulier en exposant ladite composition à un rayonnement, et plus particulièrement à des rayons UV, proche UV, visible, infrarouge ou proche-infrarouge, ou à un faisceau d'électrons, afin d'obtenir un produit réticulé qui est avantageusement une encre, un revêtement (notamment un revêtement de protection, un revêtement d'isolation électrique, un revêtement décoratif ou un revêtement réactif aux stimulus extérieurs), un matériau chargé avec des renforts fibreux ou particulaires pouvant être des nanotubes de carbone ou du graphite (notamment un mastic, une cheville chimique, une pierre artificielle, un matériau dentaire ou un composite), un adhésif, un matériau moulé, une plaque encreuse, un liant pour électrode, ou d'un objet obtenu par fabrication additive, en particulier un objet obtenu par impression 3D ou 4D.

En variante, elle peut être utilisée dans un procédé de fabrication d'un objet tridimensionnel, comprenant une étape de fabrication additive, en particulier une étape d'impression en continu ou couche par couche.

L'invention vise également l'utilisation d'un mélange d'isocyanurates (méth)acrylés selon la présente invention comme liant dans une composition polymérisable.

Enfin, le dernier objet de l'invention concerne l'utilisation d'un mélange d'isocyanurates (méth)acrylés selon la présente invention dans une composition pour la fabrication additive, en particulier pour l'impression d'un objet 3D ou 4D.

### EXEMPLES

L'invention sera mieux comprise à la lumière des exemples suivants, qui sont donnés à titre purement illustratif et n'ont pas pour but de limiter la portée de l'invention, définie par les revendications annexées.

### Matières premières

Les matières premières suivantes ont été utilisées dans les exemples :

**[Table 1]**

| **Abréviation** | **Nom chimique** | **Fonction** | **Fournisseur** |
|---|---|---|---|
| THEIC | Tris(2-hydroxyéthyl) isocyanurate | Composant c) | Jining Jianbang |
| NPG | Néopentyl glycol | Composant c) | Perstorp |
| AA | Acide acrylique | Composant b) | Arkema |
| Succ | Acide succinique | Composant a) | Roquette |
| Ad | Acide adipique | Composant a) | Solvay |
| Seb | Acid sébacique | Composant a) | Arkema |
| Cek | Acide cékanoïque (isononanoïque) | Composant b) | Brenntag |
| HQ | Hydroquinone | Inhibiteur | Solvay |
| PTZ | Phénothiazine | Inhibiteur | IMCD |
| Tol | Toluène | Solvant | Total |
| Hept | n-Heptane | Solvant | Total |
| AMS | Acide méthane sulfonique (solution aqueuse à 70 % en poids | Catalyseur | Arkema |
| SpeedCure 73 | 2-hydroxy-2-methyl-1-phenylpropanone | Photoinitiateur | Arkema |

### Méthodes

Les méthodes suivantes ont été utilisées dans la présente demande :

### Teneur en THAICT(M)A (ou THEICT(M)A)

La teneur en THAICT(M)A (ou THEICT(M)A) est calculée à partir de la probabilité qu'une (méth)acrylation se produise 3 fois sur la même molécule de THAIC (ou THEIC), cela à partir des fonctions (méth)acryliques disponibles comme fraction de la totalité des fonctions acides mises en jeu.

### Fonctionnalité en (méth)acrylate

La fonctionnalité en (méth)acrylate est calculée à partir de l'allongement moyen n, la structure globale étant représentée par la formule suivante :

[Chem 7] **(MA)₂ - THAIC** - **(DA - THAIC-MA)n - MA**

avec :
MA : résidu de monoacide du composant (b) (notamment acide (méth)acrylique et/ou acide cékanoïque),
DA : résidu de diacide du composant (a) (notamment acide succinique, acide adipique et/ou acide sébacique) ;
THAIC : résidu de THAIC (ou THEIC)
Pour n = 0, on retrouve bien la structure du triester du THAIC représenté par la formule suivante :

   [Chem 8] **(MA)₂ - THAIC** - **MA**

Pour une conversion supposée totale des fonctions hydroxyle du THAIC, l'allongement moyen du polyester est lié au ratio molaire polyacide / polyol = (a) / (c), selon l'équation suivante : [Math 1] $\left(a\right) / \left(c\right) = n / \left(n+1\right) \to n = r / \left(1-r\right) avec r = \left(a\right) / \left(c\right)$

La fonctionnalité totale f_{TOT} comme nombre d'extrémités monoacide par molécule moyenne (structure globale) est : f_{TOT} = n + 3

La fonctionnalité (méth)acrylate f_{ACR} est calculée selon l'équation suivante : [Math 2] ${f}_{ACR} = {f}_{TOT} . {x}_{ACR}$ avec x_{ACR} = fraction molaire d'acide (méth)acrylique dans le composant (b) (correspondant au ratio du nombre de moles d'acide (méth)acrylique sur le nombre de moles total du composant (b))

La fonctionnalité (méth)acrylate f_{ACR} peut donc être calculée selon l'équation suivante : [Math 3] ${f}_{ACR} = \left[\left(r/\left(1-r\right)\right)+3\right] . {x}_{ACR}$

### Coloration

L'indice de coloration APHA (American Public Health Association) est défini par une gamme étalon de solutions de référence, de colorations croissantes. Suivant la norme ISO6271, les indices APHA sont attribués de 10 à 500 à des solutions de concentration connues d'hexachloroplatinate de potassium dans l'eau, et correspondants à une quantité en mg de platine par ml de solution.

### Viscosité

La viscosité est mesurée selon la méthode Noury. Le temps de parcours d'une bille d'acier soumise à sa gravité dans le liquide à caractériser est mesuré. La méthode AFNOR XP.T51-213 spécifie la géométrie du contenant, le diamètre de la bille (2 mm) et le parcours de celle-ci (104 mm). Dans ces conditions, la viscosité dynamique est proportionnelle au temps de parcours de la bille, avec un temps de parcours de 1 seconde correspondant à une viscosité de 0,1 Pa.s.

### Indice de recristallisation

On observe (pendant 6 jours et à intervalles de temps réguliers) en microscopie optique un échantillon de produit déposé sur la lame et ensemencé par un cristal de THEICTA. On attribue un indice comparatif (de 0 à 5) :
- aucune propagation de la cristallisation : 0
- propagation totale de la cristallisation à l'ensemble de l'échantillon : 5

### Température de transition vitreuse (Tα)

### Préparation des films :

On mélange 96 % en poids de produit à tester avec 4 % en poids de photoinitiateur (SpeedCure 73), puis on applique ce mélange sur plaque de verre avec un filmographe d'épaisseur 150 µm.

Les films obtenus sont réticulés sous lampe Fusion^{®} au mercure (UV-Hg) *(voir ci-dessous test de vitesse de réticulation).* Les films ainsi réticulés sont décollés du support et placés entre 2 plaques de verre et subissent un recuit par 5 passages sous la même lampe UV à une vitesse de 5 m/min.

### Analyse mécanique dynamique :

Les films sont testés en AMD au moyen d'un appareil RSAII (Rheometrics^{®})
Sollicitation par traction, à une fréquence de 1Hz
Rampe de température : -50°C à 300°C, à une vitesse de 3°C/min

### Indice d'Acide (IA)

L'indice d'acide d'un produit est exprimé en milligrammes d'équivalent KOH par gramme de produit à caractériser. Pour cela, on effectue un dosage acide-base dans les conditions suivantes : une masse exacte m de produit (approximativement 10 grammes) est dissoute dans 50 ml d'un mélange toluène/éthanol (2 vol/1 vol). Après dissolution complète, le dosage est effectué avec une solution méthanolique d'hydroxyde de potassium de normalité N (Eq / 1) d'environ 0,1 Eq/litre. Le point équivalent est détecté par une électrode combinée contrôlant une burette automatique (titrateur automatique 716 DMS Titrino^{®} de Metrohm) qui délivre alors un volume équivalent VE. Après avoir effectué un essai à blanc (50 ml du mélange toluène / éthanol (2 vol / 1 vol) seul) qui permet la détermination du volume équivalent VB, l'indice d'acide (IA) est calculé par l'équation suivante : [Math 4] $IA = \left[\left(VE-VB\right). N .56,1\right] / m$ avec VE et VB exprimés en ml, N en Eq / litre et m en grammes.

### Réactivité sous lampe Fusion^{®} au mercure (UV-Hg)

Les formulations sont appliquées en film de 12 µm sur carte contraste "Form 1B Penoparc chart » de Leneta^{®}, puis réticulées avec une lampe Fusion^{®} au mercure d'irradiation de 120 W/cm². La vitesse minimum de passage sous la lampe (en m/min) nécessaire à l'obtention d'un film sec au toucher est mesurée.

### Flexibilité

Les formulations sont appliquées en film de 100 µm sur plaque d'acier souple d'épaisseur 25/10 mm, puis réticulées avec une lampe Fusion^{®} au mercure d'irradiation de 120 W/cm² à une vitesse de 10 m/min (2 passes). Après 24 heures de post-réticulation à 23°C, la plaque d'acier revêtue est recourbée sur les mandrins cylindriques. La flexibilité est la valeur (en mm) du plus petit rayon de courbure qui peut être appliqué au revêtement avant que celui-ci ne craque ou ne soit pelé de son support.

### Dureté Persoz

Les formulations sont appliquées en film de 100 µm sur plaque de verre, puis réticulées avec une lampe Fusion^{®} au mercure d'irradiation de 120 W/cm² à une vitesse de 10 m/min (2 passes). Après 24 heures de post-réticulation à 23°C, la dureté est déterminée par le nombre d'oscillations avant amortissement (celui-ci diminuant de 12° à 4° d'amplitude) d'un pendule en contact avec la plaque de verre revêtue.

### Résistance à l'acétone

Les formulations sont appliquées en film de 12 µm sur plaque de verre, puis réticulées avec une lampe Fusion^{®} au mercure d'irradiation de 120 W/cm² à une vitesse de 10 m/min (2 passes). Après 24 heures de post-réticulation à 23°C, le revêtement est frotté avec un chiffon imbibé d'acétone. La résistance à l'acétone est le temps (en secondes) mis pour que le revêtement soit pelé du support et/ou soit désagrégé.

### Résistance à la tâche

Les formulations sont appliquées en film de 12 µm sur une carte contraste « Leneta », puis reticulées sous lampe Fusion^{®} au mercure d'irradiation de 120 W/cm² à une vitesse de 10 m/min (2 passes). Après 24 heures de post-réticulation à 23°C, on place des disques de papier absorbant sur la carte et on y dépose du café, du parfum (2ml) et de l'iode (3 gouttes).

Après 12 heures de mise en contact, on effectue une évaluation qualitative des tâches après retrait des disques et nettoyage de la surface à l'eau :
0 : pas de trace, jusqu'à 5 : marque très importante.

### Exemple 1 : Procédé de préparation d'un mélange d'isocyanurates selon l'invention

Dans un réacteur de 1 litre équipé d'une ancre, d'un Dean-Stark, d'un bullage d'air (débit = 0,5 litre / heure) et d'un thermomètre, on charge successivement les matières premières suivantes :
Le THEIC (composant (c) : 261,0 g, 1,000 mole), l'AA (composant (b) : 202,8 g, 2,817 mole), le solvant (mélange de Hept (82 g) et Tol (328 g), c'est-à-dire une quantité globale de solvant de 40 % massique par rapport à la masse totale chargée, ce mélange solvant étant composé de 80 % de toluène et 20 % d'heptane), AMS (7,830 g, 3% massique par rapport au polyol), HQ (6,084 g, 3% massique par rapport à l'AA) et PTZ (0,008 g, 40 ppm par rapport à l'AA). Ce mélange est chauffé à 60 °C pendant une heure jusqu'à ce que l'IA (acidité faible correspondant aux groupements acide carboxylique non-réagis) atteigne une valeur inférieure à 60 mg KOH/g, soit une conversion des fonctions acide carboxylique d'environ 80 %.

On ajoute alors l'Ad (composant (a) : 24,333 g, 0,167 mole), et le mélange réactionnel est chauffé de nouveau à reflux jusqu'à ce que l'indice d'acide résiduel atteigne une valeur inférieure à 20 mg KOH/g et reste quasi constant (ΔIA pendant une heure < 0,1 mg KOH/g de décroissance).

A la fin de la réaction de polyestérification, environ 50 ml d'eau ont été distillés, ce qui correspond à une conversion de 95 % des groupements COOH. On récupère un mélange réactionnel clair (sans turbidité) d'aspect brunâtre. La masse volumique est alors ajustée à 0,95 g/l par ajout du même mélange de toluène et d'heptane (ratio massique Tol / Hept = 80 / 20).

Cette phase organique est neutralisée par ajout de 40 g de solution de NaOH (solution aqueuse à 25 % massique). La température est d'environ 50 °C, le temps d'agitation de 2 minutes, et le temps de décantation d'une heure. La phase organique est ensuite lavée 3 fois par 30 g de solution de NaOH (solution aqueuse à 25 % massique). La température est d'environ 50 °C, le temps d'agitation de 2 minutes, et le temps de décantation d'une heure, sauf pour le dernier lavage qui requiert 15 minutes de temps d'agitation. On procède ensuite à 2 lavages avec 30 g d'eau à une température d'environ 55 °C, un temps d'agitation de 5 minutes, et un temps de décantation d'une heure. La phase organique ainsi purifiée est alors distillée sous vide (4 heures à 95°C sous une pression de 0,01 MPa) afin d'en extraire les solvants (toluène et n-heptane).

### Exemples 2-3 et Exemples comparatifs

On conserve le même procédé que dans l'exemple 1 précédemment décrit, en remplaçant, pour les réactifs, les composés (a), (b) et (c) par les quantités (en moles) du tableau des exemples ci-dessous. La quantité globale de solvant est maintenue à 40 % massique de la charge totale, à l'aide d'un mélange toluène + n-heptane (Tol / Hept = 80 / 20 massique). Le catalyseur AMS est maintenu au ratio massique de 3 % par rapport au polyol. Les inhibiteurs sont quant à eux maintenus à 3 % massique pour l'HQ et 40 ppm pour la PTZ, tous deux étant rapportés à l'acide acrylique.

On procède de même pour le critère de conversion avant ajout du/des polyacide(s), ainsi que pour la conversion totale finale. Les étapes d'ajustement de la densité, de neutralisation, de lavages et de distillation du solvant sont identiques.

Les quantités de réactifs pour chaque exemple sont détaillées dans le tableau ci-dessous :

**[Table 2]**

| | | **THEICTA** | **Cékanoates** | | **Polyester à base de THEIC** | | | **Exemple 9 de** JP94081782 |
|---|---|---|---|---|---|---|---|---|
| **Composant** | **Produit** | **Comp 1** | **Comp 2** | **Comp 3** | **Ex 1** | **Ex 2** | **Ex 3** | **Comp 4** |
| **(c)** | **THEIC** | 1,000 | 1,000 | 1,000 | 1,000 | 1,000 | 1,000 | 1,000 |
| | **NPG** | 0,000 | 0,000 | 0,000 | 0,000 | 0,000 | 0,000 | 1,000 |
| **(b)** | **AA** | 3,292 | 2,800 | 3,050 | 2,817 | 2,817 | 2,583 | 2,000 |
| | **Cek** | 0,000 | 0,500 | 0,250 | 0,000 | 0,000 | 0,250 | 0,000 |
| **(a)** | **Succ** | 0,000 | 0,000 | 0,000 | 0,000 | 0,056 | 0,058 | 0,000 |
| | **Ad** | 0,000 | 0,000 | 0,000 | 0,167 | 0,056 | 0,00 | 1,000 |
| | **Seb** | 0,000 | 0,000 | 0,000 | 0,000 | 0,056 | 0,108 | 0,000 |

| | | | | | | | | |
|---|---|---|---|---|---|---|---|---|
| **Ratio molaire groupes fonctionnels** | **(a) / (b)** | 0,000 | 0,000 | 0,000 | 0,059 | 0,059 | 0,065 | 0,500 |
| | **(a) / (c)** | 0,000 | 0,000 | 0,000 | 0,111 | 0,111 | 0,111 | 0,400 |

Les produits obtenus présentent les caractéristiques suivantes :

**[Table 3]**

| **Propriétés** | **Comp 1** | **Comp 2** | **Comp 3** | **Ex 1** | **Ex 2** | **Ex 3** | **Comp 4** |
|---|---|---|---|---|---|---|---|
| **Teneur en THEICT(M)A (% poids)** | 95,3 | 53 | 71,7 | 69,7 | 69,4 | 49,8 | 9,4 |
| **Fonctionnalité en acrylate (équiv. double liaison/mole)** | 3,00 | 2,55 | 2,77 | 3,12 | 3,12 | 2,85 | 3,67 |
| **Coloration (alpha)** | > 120 | 30 | 35 | 30 | 40 | 15 | 70 |
| **Viscosité à 50°C (mPa.s)** | 900-1100 | 630 | 730 | 2900 | 2660 | 1800 | 6500 |
| **Indice de recristallisation (0-5)** | 5 | 2 | 3 | 3 | 3 | 0 | N.D. |
| **Ta (°C)** | 265 | 164 | 180 | 202 | 192 | 148 | 39 |

### Compositions comprenant un mélange d'isocyanurates

On prépare des compositions F1-F6 en combinant un mélange d'isocyanurates tel que décrit ci-dessus avec un photoinitiateur à 20°C (les quantités sont indiquées en parties en poids dans le tableau ci-dessous)

**[Table 4]**

| **Produit** | **F1 (comp)** | **F2 (comp)** | **F3 (comp)** | **F4 (invention)** | **F5 (invention)** | **F6 (invention)** | **F6 (comp)** |
|---|---|---|---|---|---|---|---|
| **SpeedCure 73** | 4 | 4 | 4 | 4 | 4 | 4 | 4 |
| **Comp 1** | 96 | - | - | - | - | - | - |
| **Comp 2** | - | 96 | - | - | - | - | - |
| **Comp 3** | - | - | 96 | - | - | - | - |
| **Ex 1** | - | - | - | 96 | - | - | - |
| **Ex 2** | - | - | - | - | 96 | - | - |
| **Ex 3** | - | - | - | - | - | 96 | - |
| **Comp 4** | - | - | - | - | - | - | 96 |

Les propriétés applicatives des compositions sont détaillées dans le tableau ci-dessous :

**[Table 5]**

| **Propriétés** | | **F1 (comp)** | **F2 (comp)** | **F3 (comp)** | **F4 (invention)** | **F5 (invention)** | **F6 (invention)** | **F6 (comp)** |
|---|---|---|---|---|---|---|---|---|
| **Réactivité (m/min)** | | 35 | 5 | 15 | 30 | 35 | 15 | 5 |
| **Flexibilité (mm)** | | > 32 | > 32 | > 32 | > 32 | > 32 | > 32 | < 2 |
| **Dureté (s)** | | 356 | 342 | 347 | 353 | 358 | 357 | 79 |
| **Résistance à l'acétone (s)** | | 300 | 300 | 300 | 300 | 300 | 300 | 300 |
| **Résistance à la tâche (0-5)** | **café** | 0 | 0 | 0 | 0 | 0 | 0 | 3 |
| | **parfum** | 0 | 0 | 0 | 0 | 0 | 0 | 0 |
| | **iode** | 1 | 2 | 1 | 0-1 | 0-1 | 2 | 5 |

On voit d'emblée que l'exemple comparatif F6 (tel que décrit dans JP94081782) n'appartient pas au domaine de l'invention. Le taux de THEICTA, jouant le rôle de diluant réactif dans le polyester acrylate, est beaucoup plus faible (< 10 %) que celui des autres exemples (> 50 %), d'où une viscosité beaucoup plus importante (> 6 Pa.s par rapport aux autres valeurs toutes < 3 Pa.s) et une Tα beaucoup plus faible (< 40°C par rapport aux autres valeurs toutes > 140°C).

L'utilisation d'acide cékanoïque dans les Formulations comparatives F2-F3 pour estérifier le THEIC améliore l'indice de recristallisation, mais il entre dans la composition de l'ester final au détriment de l'acide acrylique (ce que montre l'abaissement de la fonctionnalité moyenne qui est de 3,00 équivalents de double liaison/mole pour le THEICTA, et seulement de 2,77 et 2,55 équivalents de double liaison/mole respectivement pour les formulations comparatives F3 et F2). Cette différence de structure explique la très forte diminution de la Tα et de la dureté qui sont des propriétés essentielles du THEICTA.

Les formulations F4-F6 comprenant le mélange d'isocyanurates selon l'invention montrent que l'utilisation conjointe du monoacide et de diacides permet d'atteindre un compromis de propriétés (Tα, viscosité, réactivité), tout en éradiquant le problème de la recristallisation du THEICTA.

## Revendications

1. Mélange d'isocyanurates (méth)acrylés, **caractérisé en ce qu'**il est obtenu par réaction entre :
(a) au moins un acide dicarboxylique,
(b) au moins un monomère (méth)acrylique choisi parmi l'acide acrylique, l'acide méthacrylique, leurs anhydrides et leurs mélanges, et éventuellement au moins un acide monocarboxylique en C6-C24,
(c) au moins un isocyanurate de tris(hydroxyalkyle) et éventuellement un autre polyol,
étant entendu que le ratio molaire des groupes -COOH du constituant (a) aux groupes -OH du constituant (c) est compris entre 1:4 et 1:20, de préférence entre 1:5 et 1:15, plus préférablement entre 1:6 et 1:12.

2. Mélange selon la revendication 1, **caractérisé en ce que** l'isocyanurate de tris(hydroxyalkyle) est choisi parmi l'isocyanurate de tris(2-hydroxyméthyle), l'isocyanurate de tris(2-hydroxyéthyle), l'isocyanurate de tris(2-hydroxypropyle), l'isocyanurate de tris(2-hydroxyisopropyle), l'isocyanurate de tris(3-hydroxypropyle), l'isocyanurate de tris(2-hydroxybutyle), l'isocyanurate de tris(4-hydroxybutyle), ainsi que les dérivés alkoxylés (notamment éthoxylés et/ou propoxylés) de ceux-ci ; de préférence l'isocyanurate de tris(hydroxyalkyle) est l'isocyanurate de tris(2-hydroxyéthyle).

3. Mélange selon la revendication 1 ou 2, **caractérisé en ce que** l'isocyanurate de tris(hydroxyalkyle) représente de 75 à 100%, de préférence 80 à 100%, plus préférentiellement 85 à 100%, molaire du nombre total de moles du constituant (c).

4. Mélange selon l'une quelconque des revendications 1 à 3, **caractérisé en ce que** l'acide dicarboxylique est choisi parmi :
- un acide dicarboxylique aliphatique saturé tel que l'acide adipique, l'acide sébacique, l'acide succinique, l'acide 2-méthylsuccinique, l'acide 2-éthylsuccinique, l'acide 2,2-diméthylsuccinique, l'acide 1,11-undécanedioïque, l'acide 1,12-dodécanedioïque, l'acide oxalique, l'acide malonique, l'acide 2-méthylmalonique, l'acide 2-éthylmalonique, l'acide glutarique, l'acide 3,3-diméthylglutarique, l'acide 3,3-diéthylglutarique, l'acide pimélique, l'acide subérique, l'acide azélaïque ou un dimère d'acide gras en C32-C36 ;
- un acide dicarboxylique aliphatique insaturé tel que l'acide itaconique, l'acide citraconique, l'acide mésaconique, l'acide glutaconique, l'acide muconique, l'acide fumarique ou l'acide maléique,
- un acide dicarboxylique cycloaliphatique saturé tel que l'acide cyclopentane 1,2- ou 1,3-dicarboxylique, l'acide cyclohexane 1,2-, 1,3- ou 1,4-dicarboxylique, l'acide cycloheptane 1,2-dicarboxylique, le 1,2-, 1,3 ou 1,4-bis(carboxyméthyl)cyclohexane ;
- un acide dicarboxylique cycloaliphatique insaturé tel que l'acide tétrahydrophtalique,
- un acide dicarboxylique aromatique tel que l'acide phtalique, l'acide isophtalique, l'acide téréphtalique, le bis(4-carboxyphényl)méthane ;
ainsi que les dérivés de ceux-ci (notamment les diesters ou anhydrides cycliques de ceux-ci) et leurs mélanges.

5. Mélange selon l'une quelconque des revendications 1 à 4, **caractérisé en ce que** l'acide dicarboxylique est un acide dicarboxylique aliphatique saturé, de préférence un acide dicarboxylique aliphatique saturé en C4-C10, plus préférentiellement un acide dicarboxylique choisi parmi l'acide adipique, l'acide sébacique, l'acide succinique et leurs mélanges, plus préférentiellement encore un mélange d'acide succinique et sébacique.

6. Mélange selon l'une quelconque des revendications 1 à 5, **caractérisé en ce que** le constituant (b) comprend un acide monocarboxylique, dans un ratio molaire de l'acide monocarboxylique au monomère (méth)acrylique compris entre 5:95 et 15:85.

7. Mélange selon l'une quelconque des revendications 1 à 6, **caractérisé en ce que** ledit autre polyol est choisi parmi : éthylène glycol, 1,2- ou 1,3-propanediol, 1,2-, 1,3-, 2,3- ou 1,4-butanediol, 1,5-pentanediol, 1,6-hexanediol, 3-méthyl-1,5-pentanediol, 1,10-decanediol, 1,12-dodecanediol, di-, tri- ou polyéthylène glycol, di-, tri ou polypropylène glycol, 1,4-cyclohexanediméthanol, 1,6-cyclohexanediméthanol, 1,4-cyclohexanediol, bisphénol A, bisphénol A hydrogéné, glycérol, diglycérol, tricyclodécane diméthanol, triméthylolpropane, di(triméthylolpropane), triméthyloléthane, 1,2,6-hexanetriol, 1,2,4-butanetriol, érythritol, pentaérythritol, di(pentaérythritol), néopentyl glycol, 2-butyl-2-ethyl-1,3-propanediol, 2-méthyl-1,3-propanediol, 2-méthyl-1,2-propanediol, sorbitol, mannitol, xylitol, isosorbide, isoidide, isomannide, méthyl glucoside, un polyester polyols (notamment polycaprolactone polyol), un polycarbonate polyol, un polyorganosiloxane polyol (notamment polydiméthysiloxane polyol), un polyglycérol (notamment Polyglycérol-3 (trimère de glycérol) et le décaglycérol), un polybutadiène à terminaison hydroxy, un diol dérivé d'un dimère ou trimère d'acide gras hydrogéné ou non, les dérivés alkoxylés (notamment éthoxylés et/ou propoxylés) des polyols cités ci-dessus, et des mélanges de ceux-ci, de préférence le sorbitol.

8. Mélange selon l'une quelconque des revendications 1 à 6, **caractérisé en ce que** le mélange réactionnel, en particulier le constituant (c), ne renferme pas d'autre polyol que l'isocyanurate de tris(hydroxyalkyle).

9. Mélange selon l'une quelconque des revendications 1 à 8, **caractérisé en ce qu'**il comprend du triacrylate de tris(hydroxyalkyle) isocyanurate.

10. Mélange selon l'une quelconque des revendications 1 à 9, **caractérisé en ce qu'**il comprend un composant polyester PE comprenant un mélange de composés répondant à la formule (V) suivante : 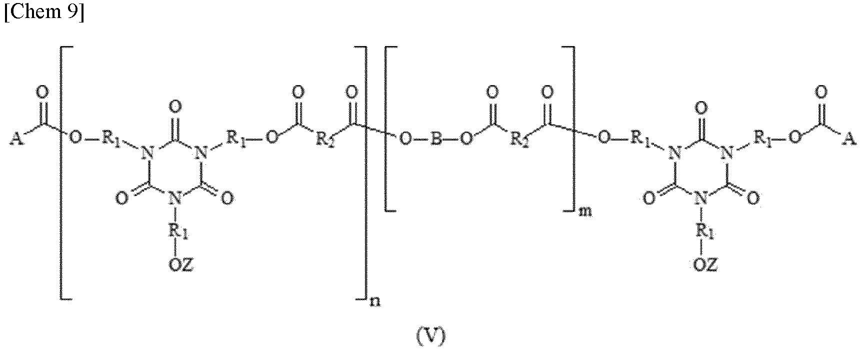 dans laquelle
chaque R₁ est indépendamment un alkylène en C2-C12 éventuellement alkoxylé ;
chaque R₂ est indépendamment le résidu d'un acide dicarboxylique ;
chaque A est indépendamment le résidu d'un acide (méth)acrylique ou d'un acide monocarboxylique, de préférence le résidu d'un acide (méth)acrylique ;
chaque B est indépendamment le résidu d'un polyol autre qu'un isocyanurate de tris(hydroxyalkyle) ;
chaque Z est indépendamment H ou -C(=O)-A ;
m et n sont des valeurs moyennes où n va de 1 à 10, de préférence n va de 1 à 2, et m va de 0 à 10, de préférence m est égal à 0.

11. Mélange selon l'une quelconque des revendications 1 à 10, **caractérisé en ce qu'**il comprend un composant polyester PE comprenant un mélange de composés répondant à la formule (VI) suivante : 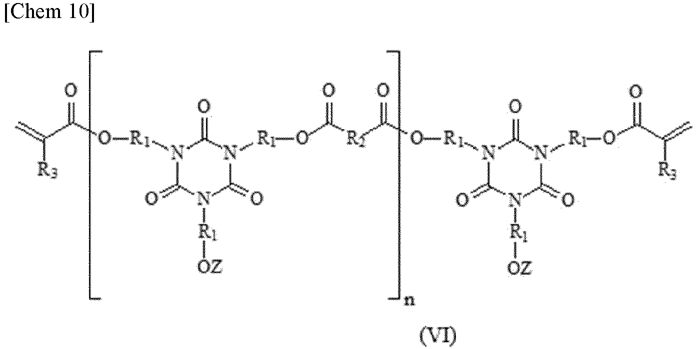 dans laquelle
chaque R₁ est indépendamment un alkylène en C2-C12 éventuellement alkoxylé ;
chaque R₂ est indépendamment le résidu d'un acide dicarboxylique ;
chaque R₃ est indépendamment H ou méthyle ;
chaque Z est indépendamment H ou -C(=O)-C(R₃)=CH₂ ;
n va de 1 à 10, de préférence n va de 1 à 2.

12. Procédé de préparation d'un mélange d'isocyanurates (méth)acrylés selon l'une quelconque des revendications 1 à 11, **caractérisé en ce qu'**il comprend les étapes suivantes :
1) faire réagir tout ou partie d'un constituant (b) comprenant au moins un monomère (méth)acrylique choisi parmi l'acide acrylique, l'acide méthacrylique, leurs anhydrides et leurs mélanges et éventuellement au moins un acide monocarboxylique en C6-C24, avec un constituant (c) comprenant au moins un isocyanurate de tris(hydroxyalkyle) et éventuellement un autre polyol P_{OH}, dans des conditions permettant d'estérifier de 50 à 95%, de préférence de 60 à 90%, plus préférentiellement de 70 à 85%, des groupes -OH du constituant (c),
2) faire réagir le mélange de l'étape 1) avec un constituant (a) comprenant au moins un acide dicarboxylique, et éventuellement avec le reste, le cas échéant, du constituant (b).

13. Composition polymérisable **caractérisée en ce qu'**elle comprend un mélange d'isocyanurates (méth)acrylés selon l'une quelconque des revendications 1 à 11 et au moins un autre composé éthyléniquement insaturé, en particulier un monomère fonctionnalisé par (méth)acrylate.

14. Composition polymérisable selon la revendication 13, **caractérisée en ce que** la composition polymérisable est une composition d'encre, de revêtement, de matériau chargé avec des renforts fibreux ou particulaires pouvant être des nanotubes de carbone ou du graphite, une composition d'adhésif, de moulage, de plaque encreuse, de liant pour électrode, ou une composition pour la fabrication additive, en particulier pour l'impression d'objets 3D ou 4D.

15. Utilisation d'un mélange d'isocyanurates (méth)acrylés selon l'une quelconque des revendications 1 à 11 comme liant dans une composition polymérisable ou dans une composition pour la fabrication additive, en particulier pour l'impression d'un objet 3D ou 4D.

## Patentansprüche

1. Mischung von (meth)acrylierten Isocyanuraten, **dadurch gekennzeichnet, dass** sie durch eine Reaktion zwischen Folgendem erhalten wird:
(a) mindestens einer Dicarbonsäure,
(b) mindestens einem (Meth)Acryl-Monomer, das aus Acrylsäure, Methacrylsäure, Anhydriden davon und Mischungen davon und gegebenenfalls mindestens einer C6-C24-Monocarbonsäure ausgewählt ist,
(c) mindestens einem Tris(hydroxyalkyl)isocyanurat und gegebenenfalls einem anderen Polyol,
mit der Maßgabe, dass das Stoffmengenverhältnis der - COOH-Gruppen von Bestandteil (a) zu den -OH-Gruppen von Bestandteil (c) zwischen 1:4 und 1:20, vorzugsweise zwischen 1:5 und 1:15, stärker bevorzugt zwischen 1:6 und 1:12, beträgt.

2. Mischung nach Anspruch 1, **dadurch gekennzeichnet, dass** das Tris(hydroxyalkyl)isocyanurat aus Tris(2-hydroxymethyl)isocyanurat, Tris(2-hydroxyethyl)isocyanurat, Tris(2-hydroxypropyl)isocyanurat, Tris(2-hydroxyisopropyl)isocyanurat, Tris(3-hydroxypropyl)isocyanurat, Tris(2-hydroxybutyl)isocyanurat, Tris(4-hydroxybutyl)isocyanurat sowie alkoxylierten (insbesondere ethoxylierten und/oder propoxylierten) Derivaten davon ausgewählt ist; das Tris(hydroxyalkyl)isocyanurat vorzugsweise Tris(2-hydroxyethyl)isocyanurat ist.

3. Mischung nach Anspruch 1 oder 2, **dadurch gekennzeichnet, dass** das Tris(hydroxyalkyl)isocyanurat 75 bis 100 Mol-%, vorzugsweise 80 bis 100 Mol-%, stärker bevorzugt 85 bis 100 Mol-%, der Gesamtmolzahl von Bestandteil (c) darstellt.

4. Mischung nach einem der Ansprüche 1 bis 3, **dadurch gekennzeichnet, dass** die Dicarbonsäure ausgewählt ist aus:
- einer gesättigten aliphatischen Dicarbonsäure, wie Adipinsäure, Sebacinsäure, Bernsteinsäure, 2-Methylbernsteinsäure, 2-Ethylbernsteinsäure, 2,2-Dimethylbernsteinsäure, 1,11-Undecandicarbonsäure, 1,12-Dodecandicarbonsäure, Oxalsäure, Malonsäure, 2-Methylmalonsäure, 2-Ethylmalonsäure, Glutarsäure, 3,3-Dimethylglutarsäure, 3,3-Dilethylglutarsäure, Pimelinsäure, Korksäure, Azelainsäure oder einem C32-C36-Fettsäuredimer;
- einer ungesättigten aliphatischen Dicarbonsäure, wie Itaconsäure, Citraconsäure, Mesaconsäure, Glutaconsäure, Muconsäure, Fumarsäure oder Maleinsäure,
- einer gesättigten cycloaliphatischen Dicarbonsäure, wie Cyclopentan-1,2- oder -1,3-dicarbonsäure, Cyclohexan-1,2-, -1,3- oder -1,4-dicarbonsäure, Cycloheptan-1,2-dicarbonsäure, 1,2-, 1,3- oder 1,4-Bis(carboxymethyl)cyclohexan;
- einer ungesättigten cycloaliphatischen Dicarbonsäure, wie Tetrahydrophthalsäure,
- einer aromatischen Dicarbonsäure, wie Phthalsäure, Isophthalsäure, Terephthalsäure, Bis(4-carboxyphenyl)methan;
sowie Derivaten davon (insbesondere Diestern oder cyclischen Anhydriden davon) und Mischungen davon.

5. Mischung nach einem der Ansprüche 1 bis 4, **dadurch gekennzeichnet, dass** es sich bei der Dicarbonsäure um eine gesättigte aliphatische Dicarbonsäure, vorzugsweise eine gesättigte aliphatische C4-C10-Dicarbonsäure, stärker bevorzugt eine Dicarbonsäure, die aus Adipinsäure, Sebacinsäure, Bernsteinsäure und Mischungen davon, noch stärker bevorzugt um eine Mischung von Bernsteinsäure und Sebacinsäure, handelt.

6. Mischung nach einem der Ansprüche 1 bis 5, **dadurch gekennzeichnet, dass** Bestandteil (b) eine Monocarbonsäure mit einem Stoffmengenverhältnis der Monocarbonsäure zum (Meth)Acryl-Monomer zwischen 5:95 und 15:85 umfasst.

7. Mischung nach einem der Ansprüche 1 bis 6, **dadurch gekennzeichnet, dass** das andere Polyol ausgewählt ist aus: Ethylenglycol, 1,2- oder 1,3-Propandiol, 1,2-, 1,3-, 2,3- oder 1,4-Butandiol, 1,5-Pentandiol, 1,6-Hexandiol, 3-Methyl-1,5-pentandiol, 1,10-Decandiol, 1,12-Dodecandiol, Di-, Tri- oder Polyethylenglycol, Di-, Tri oder Polypropylenglycol, 1,4-Cyclohexandimethanol, 1,6-Cyclohexandimethanol, 1,4-Cyclohexandiol, Bisphenol A, hydriertem Bisphenol A, Glycerin, Diglycerin, Tricyclodecandimethanol, Trimethylolpropan, Di(trimethylolpropan), Trimethylolethan, 1,2,6-Hexantriol, 1,2,4-Butantriol, Erythrit, Pentaerythrit, Di(pentaerythrit), Neopentylglycol, 2-Butyl-2-ethyl-1,3-propandiol, 2-Methyl-1,3-propandiol, 2-Methyl-1,2-propandiol, Sorbit, Mannit, Xylit, Isosorbid, Isoidid, Isomannid, Methylglucosid, einem Polyesterpolyol (insbesondere Polycaprolactonpolyol), einem Polycarbonatpolyol, einem Polyorganosiloxanpolyol (insbesondere Polydimethysiloxanpolyol), einem Polyglycerin (insbesondere Polyglycerin-3 (Glycerintrimer) und Decaglycerin), einem Polybutadien mit Hydroxy-Endgruppen, einem Diol, das von einem hydrierten oder nicht hydrierten Fettsäuredimer oder - trimer, alkoxylierten (insbesondere ethoxylierten und/oder propoxylierten) Derivaten der obengenannten Polyole und Mischungen davon, vorzugsweise Sorbit.

8. Mischung nach einem der Ansprüche 1 bis 6, **dadurch gekennzeichnet, dass** die Reaktionsmischung, insbesondere Bestandteil (c), kein anderes Polyol als ein Tris(hydroxyalkyl)isocyanurat enthält.

9. Mischung nach einem der Ansprüche 1 bis 8, **dadurch gekennzeichnet, dass** sie ein Tris(hydroxyalkyl)isocyanurattriacrylat umfasst.

10. Mischung nach einem der Ansprüche 1 bis 9, **dadurch gekennzeichnet, dass** sie eine Polyesterkomponente PE umfasst, die eine Mischung von Verbindungen umfasst, die der folgenden Formel (V) entsprechen: 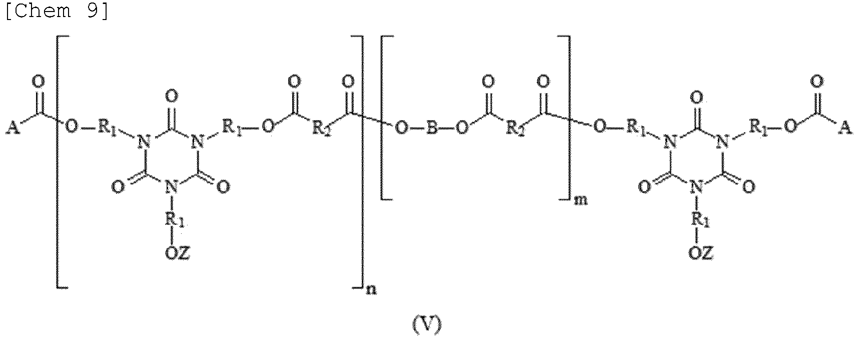 wobei
jeder R₁ unabhängig ein gegebenenfalls alkoxyliertes C2-C12-Alkylen ist;
jeder R₂ unabhängig der Rest einer Dicarbonsäure ist;
jedes A unabhängig der Rest einer (Meth)Acrylsäure oder einer Monocarbonsäure, vorzugsweise der Rest einer (Meth)Acrylsäure, ist;
jedes B unabhängig der Rest eines von einem Tris(hydroxyalkyl)isocyanurat verschiedenen Polyols ist;
jedes Z unabhängig H oder -C(=O)-A ist;
m und n Mittelwerte sind, wobei n 1 bis 10 beträgt, n vorzugsweise 1 bis 2 beträgt und m 0 bis 10 beträgt, m vorzugsweise gleich 0 ist.

11. Mischung nach einem der Ansprüche 1 bis 10, **dadurch gekennzeichnet, dass** sie eine Polyesterkomponente PE umfasst, die eine Mischung von Verbindungen umfasst, die der folgenden Formel (VI) entsprechen: 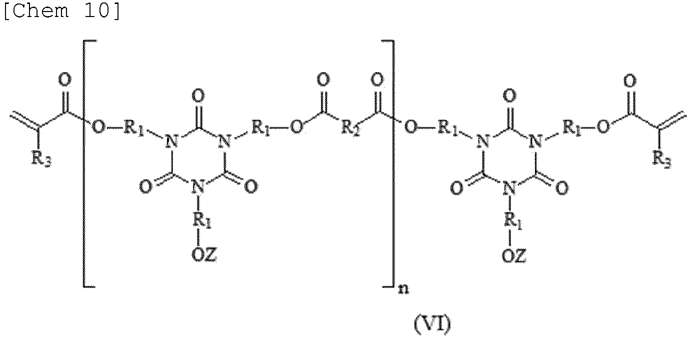 wobei
jeder R₁ unabhängig ein gegebenenfalls alkoxyliertes C2-C12-Alkylen ist;
jeder R₂ unabhängig der Rest einer Dicarbonsäure ist;
jeder R₃ unabhängig H oder Methyl ist;
jedes Z unabhängig H oder -C(=O)-C(R₃)=CH₂ ist;
n 1 bis 10 beträgt, n vorzugsweise 1 bis 2 beträgt.

12. Verfahren zur Herstellung einer Mischung von (meth)acrylierten Isocyanuraten nach einem der Ansprüche 1 bis 11, **dadurch gekennzeichnet, dass** es die folgenden Schritte umfasst:
1) das Umsetzen des gesamten Bestandteils (b) oder eines Teils davon, der mindestens ein (Meth)Acryl-Monomer umfasst, das aus Acrylsäure, Methacrylsäure, Anhydriden davon und Mischungen davon und gegebenenfalls einer C6-C24-Monocarbonsäure ausgewählt ist, mit einem Bestandteil (c), der mindestens ein Tris(hydroxyalkyl)isocyanurat und gegebenenfalls ein anderes Polyol P_{OH} umfasst, unter Bedingungen, die ein Verestern von 50 bis 95 %, vorzugsweise von 60 bis 90 %, stärker bevorzugt von 70 bis 85 % der -OH-Gruppen von Bestandteil (c) ermöglichen,
2) das Umsetzen der Mischung von Schritt 1) mit einem Bestandteil (a), der mindestens eine Dicarbonsäure umfasst, und gegebenenfalls mit dem Rest, soweit erforderlich, von Bestandteil (b).

13. Polymerisierbare Zusammensetzung, **dadurch gekennzeichnet, dass** sie eine Mischung von (meth)acrylierten Isocyanuraten nach einem der Ansprüche 1 bis 11 und mindestens eine andere ethylenisch ungesättigte Verbindung, insbesondere ein (Meth)Acrylat-funktionalisiertes Monomer, umfasst.

14. Polymerisierbare Zusammensetzung nach Anspruch 13, **dadurch gekennzeichnet, dass** es sich bei der polymerisierbaren Zusammensetzung um eine Tinten-, eine Beschichtungs-, eine mit Faser- oder Partikelverstärkungen, wobei es sich um Kohlenstoff-Nanoröhrchen oder Graphit handeln kann, gefüllte Materialzusammensetzung, eine Klebstoff-, eine Formpress-, eine Farbkissen-, eine Elektrodenbindemittel-Zusammensetzung oder eine Zusammensetzung zur additiven Fertigung, insbesondere zum Drucken von 3D- oder 4D-Gegenständen, handelt.

15. Verwendung einer Mischung von (meth)acrylierten Isocyanuraten nach einem der Ansprüche 1 bis 11 als Bindemittel in einer polymerisierbaren Zusammensetzung oder in einer Zusammensetzung zur additiven Fertigung, insbesondere zum Drucken eines 3D- oder 4D-Gegenstandes.

## Claims

1. Mixture of (meth)acrylated isocyanurates, **characterized in that** it is obtained by reaction between:
(a) at least one dicarboxylic acid,
(b) at least one (meth)acrylic monomer selected from acrylic acid, methacrylic acid, anhydrides thereof, and mixtures thereof, and optionally at least one C₆-C₂₄ monocarboxylic acid,
(c) at least one tris(hydroxyalkyl) isocyanurate and optionally another polyol,
it being understood that the molar ratio of -COOH groups of constituent (a) to -OH groups of constituent (c) is between 1:4 and 1:20, preferably between 1:5 and 1:15, more preferably between 1:6 and 1:12.

2. Mixture according to Claim 1, **characterized in that** the tris(hydroxyalkyl) isocyanurate is selected from tris(2-hydroxymethyl) isocyanurate, tris(2-hydroxyethyl) isocyanurate, tris(2-hydroxypropyl) isocyanurate, tris(2-hydroxyisopropyl) isocyanurate, tris(3-hydroxypropyl) isocyanurate, tris(2-hydroxybutyl) isocyanurate, tris(4-hydroxybutyl) isocyanurate, and also alkoxylated (in particular ethoxylated and/or propoxylated) derivatives thereof; preferably, the tris(hydroxyalkyl) isocyanurate is tris(2-hydroxyethyl) isocyanurate.

3. Mixture according to Claim 1 or 2, **characterized in that** the tris(hydroxyalkyl) isocyanurate represents from 75 to 100 mol%, preferably 80 to 100 mol%, more preferably 85 to 100 mol%, of the total number of moles of constituent (c).

4. Mixture according to any one of Claims 1 to 3, **characterized in that** the dicarboxylic acid is selected from:
- a saturated aliphatic dicarboxylic acid, such as adipic acid, sebacic acid, succinic acid, 2-methylsuccinic acid, 2-ethylsuccinic acid, 2,2-dimethylsuccinic acid, 1,11-undecanedioic acid, 1,12-dodecanedioic acid, oxalic acid, malonic acid, 2-methylmalonic acid, 2-ethylmalonic acid, glutaric acid, 3,3-dimethylglutaric acid, 3,3-diethylglutaric acid, pimelic acid, suberic acid, azelaic acid or a C₃₂-C₃₆ fatty acid dimer;
- an unsaturated aliphatic dicarboxylic acid, such as itaconic acid, citraconic acid, mesaconic acid, glutaconic acid, muconic acid, fumaric acid or maleic acid;
- a saturated cycloaliphatic dicarboxylic acid, such as cyclopentane-1,2- or -1,3-dicarboxylic acid, cyclohexane-1,2-, -1,3- or -1,4-dicarboxylic acid, cycloheptane-1,2-dicarboxylic acid or 1,2-, 1,3- or 1,4-bis(carboxymethyl)cyclohexane;
- an unsaturated cycloaliphatic dicarboxylic acid, such as tetrahydrophthalic acid;
- an aromatic dicarboxylic acid, such as phthalic acid, isophthalic acid, terephthalic acid or bis(4-carboxyphenyl)methane;
and also the derivatives of these (in particular the diesters or cyclic anhydrides of these) and their mixtures.

5. Mixture according to any one of Claims 1 to 4, **characterized in that** the dicarboxylic acid is a saturated aliphatic dicarboxylic acid, preferably a saturated C₄-C₁₀ aliphatic dicarboxylic acid, more preferably a dicarboxylic acid selected from adipic acid, sebacic acid, succinic acid, and mixtures thereof, even more preferably a mixture of succinic and sebacic acid.

6. Mixture according to any one of Claims 1 to 5, **characterized in that** constituent (b) comprises a monocarboxylic acid in a molar ratio of monocarboxylic acid to (meth)acrylic monomer of between 5:95 and 15:85.

7. Mixture according to any one of Claims 1 to 6, **characterized in that** said other polyol is selected from: ethylene glycol, propane-1,2- or -1,3-diol, butane-1,2-, -1,3- -2,3- or -1,4-diol, pentane-1,5-diol, hexane-1,6-diol, 3-methylpentane-1,5-diol, decane-1,10-diol, dodecane-1,12-diol, di-, tri- or polyethylene glycol, di- , tri- or polypropylene glycol, cyclohexane-1,4-dimethanol, cyclohexane-1,6-dimethanol, cyclohexane-1,4-diol, bisphenol A, hydrogenated bisphenol A, glycerol, diglycerol, tricyclodecanedimethanol, trimethylolpropane, di(trimethylolpropane), trimethylolethane, hexane-1,2,6-triol, butane-1,2,4-triol, erythritol, pentaerythritol, di(pentaerythritol), neopentyl glycol, 2-butyl-2-ethylpropane-1,3-diol, 2-methylpropane-1,3-diol, 2-methylpropane-1,2-diol, sorbitol, mannitol, xylitol, isosorbide, isoidide, isomannide, methylglucoside, a polyester polyol (in particular polycaprolactone polyol), a polycarbonate polyol, a polyorganosiloxane polyol (in particular polydimethysiloxane polyol), a polyglycerol (in particular polyglycerol-3 (glycerol trimer) and decaglycerol), a hydroxy-terminated polybutadiene, a diol derived from a hydrogenated or non-hydrogenated dimer or trimer fatty acid, alkoxylated (in particular ethoxylated and/or propoxylated) derivatives of the polyols recited above, and mixtures thereof, preferably sorbitol.

8. Mixture according to any one of Claims 1 to 6, **characterized in that** the reaction mixture, in particular constituent (c), does not contain any polyol other than tris(hydroxyalkyl) isocyanurate.

9. Mixture according to any one of Claims 1 to 8, **characterized in that** it comprises tris(hydroxyalkyl) isocyanurate triacrylate.

10. Mixture according to any one of Claims 1 to 9, **characterized in that** it comprises a polyester component PE comprising a mixture of compounds corresponding to formula (V) below: 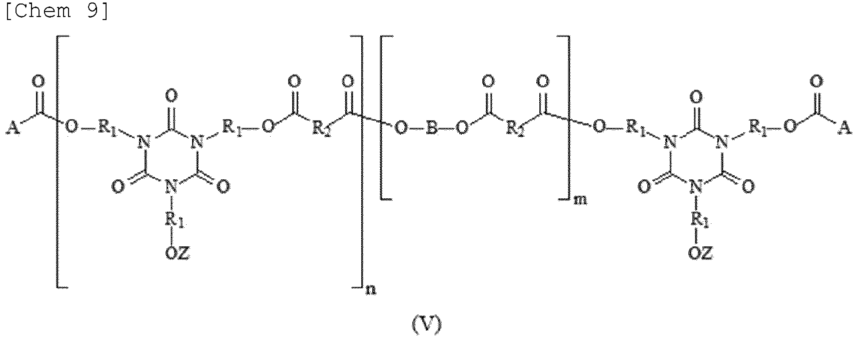 in which:
each R₁ is independently an optionally alkoxylated C₂-C₁₂ alkylene;
each R₂ is independently a dicarboxylic acid residue;
each A is independently a (meth)acrylic acid residue or
a monocarboxylic acid residue, preferably a (meth)acrylic acid residue;
each B is independently the residue of a polyol other than a tris(hydroxyalkyl) isocyanurate;
each Z is independently H or -C(=O)-A;
m and n are average values where n ranges from 1 to 10,
preferably n ranges from 1 to 2, and m ranges from 0 to 10; preferably, m is equal to 0.

11. Mixture according to any one of Claims 1 to 10, **characterized in that** it comprises a polyester component PE comprising a mixture of compounds corresponding to formula (VI) below: 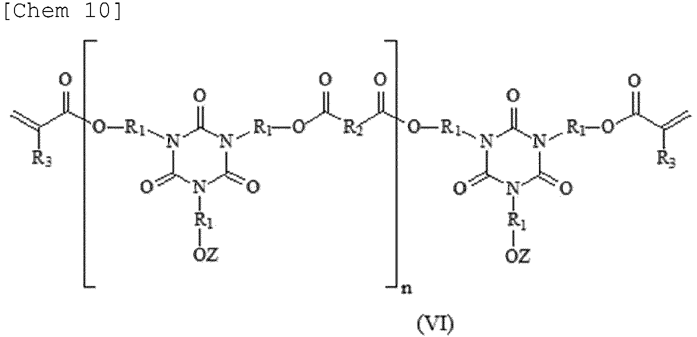 in which:
each R₁ is independently an optionally alkoxylated C₂-C₁₂ alkylene;
each R₂ is independently a dicarboxylic acid residue;
each R₃ is independently H or methyl;
each Z is independently H or -C(=O)-C(R₃)=CH₂;
n ranges from 1 to 10; preferably, n ranges from 1 to 2.

12. Process for preparing a mixture of (meth)acrylated isocyanurates according to any one of Claims 1-11, **characterized in that** it comprises the following steps:
1) reacting all or part of a constituent (b) comprising at least one (meth)acrylic monomer selected from acrylic acid, methacrylic acid, anhydrides thereof, and mixtures thereof and optionally at least one C₆-C₂₄ monocarboxylic acid with a constituent (c) comprising at least one tris(hydroxyalkyl) isocyanurate and optionally another polyol P_{OH} under conditions that permit the esterification of from 50 to 95%, preferably from 60 to 90%, more preferably from 70 to 85%, of the OH groups of constituent (c),
2) reacting the mixture from step 1) with a constituent (a) comprising at least one dicarboxylic acid and optionally with any residual amount of constituent (b).

13. Polymerizable composition **characterized in that** it comprises a mixture of (meth)acrylated isocyanurates according to any one of Claims 1-11 and at least one other ethylenically unsaturated compound, in particular a (meth)acrylate-functionalized monomer.

14. Polymerizable composition according to Claim 13, **characterized in that** the polymerizable composition is an ink composition, a coating composition, a material filled with fibrous or particulate reinforcements, which may be carbon nanotubes or graphite, an adhesive composition, moulding composition, ink plate composition, electrode binder composition, or a composition for additive manufacturing, in particular for the 3D or 4D printing of articles.

15. Use of a mixture of (meth)acrylated isocyanurates according to any one of Claims 1-11 as binder in a polymerizable composition or in a composition for additive manufacturing, in particular for printing a 3D or 4D article.
